(19) Europäisches Patentamt
European Patent Office
Office européen des brevets

(11) **EP 3 949 955 A1**

(12) **EUROPEAN PATENT APPLICATION**
published in accordance with Art. 153(4) EPC


(43) Date of publication:
**09.02.2022 Bulletin 2022/06**

(21) Application number: **20784346.7**

(22) Date of filing: **01.04.2020**

(51) International Patent Classification (IPC):
***A61K 9/48*** *(2006.01)* ***A61K 9/28*** *(2006.01)*
***A61K 31/4439*** *(2006.01)* ***A61P 1/04*** *(2006.01)*

(52) Cooperative Patent Classification (CPC):
**A61K 9/28; A61K 9/48; A61K 31/4439; A61P 1/04**

(86) International application number:
**PCT/KR2020/004474**

(87) International publication number:
**WO 2020/204609 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2019 KR 20190038380**



(71) Applicant: **Hanmi Pharm. Co., Ltd.**
**Hwaseong-si, Gyeonggi-do 18536 (KR)**

(72) Inventors:
- **CHO, Hyuk Jun**
  **Uiwang-si, Gyeonggi-do 16036 (KR)**
- **KWON, Taek Kwan**
  **Hwaseong-si, Gyeonggi-do 18466 (KR)**
- **IM, Ho Taek**
  **Paju-si, Gyeonggi-do 10892 (KR)**
- **KIM, Yong Il**
  **Gwacheon-si, Gyeonggi-do 13835 (KR)**
- **JUNG, Jin A**
  **Seoul 06904 (KR)**
- **SON, Han Kil**
  **Seoul 06370 (KR)**
- **HONG, Sung Hee**
  **Seoul 05668 (KR)**

(74) Representative: **Brevalex**
**95, rue d'Amsterdam**
**75378 Paris Cedex 8 (FR)**


(54) **PHARMACEUTICAL COMPOSITION COMPRISING ESOMEPRAZOLE OR PHARMACEUTICALLY ACCEPTABLE SALT THEREOF AND HAVING DOUBLE-RELEASE PROFILE**


(57) The present disclosure relates to a pharmaceutical composition comprising esomeprazole or a pharmaceutically acceptable salt thereof and with a dual release profile, and particularly to a pharmaceutical composition which exhibits a dual release profile of immediate release and sustained release so that long-term efficacy can be sustained.

The pharmaceutical composition comprising esomeprazole or a pharmaceutically acceptable salt thereof and with a dual release profile, according to the present disclosure, can secure bioavailability equivalent to that of existing esomeprazole immediate-release/enteric-release formulations, and can maintain long-term efficacy due to the dual release profile thereof even when administered once a day, thus preventing the occurrence of nocturnal acid breakthrough, and accordingly, can be effectively used as a therapeutic agent for nocturnal acid breakthrough.


FIG. 2

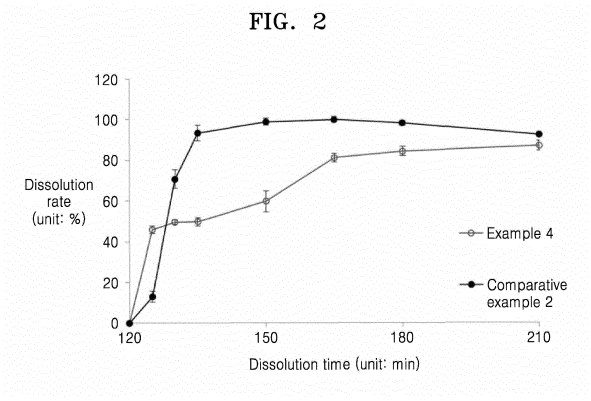


EP 3 949 955 A1

## Description

TECHNICAL FIELD

The present disclosure relates to a pharmaceutical composition comprising esomeprazole or a pharmaceutically acceptable salt thereof and having a dual release profile. More specifically, the present disclosure relates to a pharmaceutical composition comprising esomeprazole or a pharmaceutically acceptable salt thereof and having a dual release profile, for use in preventing or inhibiting nocturnal acid breakthrough.

BACKGROUND ART

For prevention or treatment of diseases associated with hypersecretion of gastric acid, including gastroesophageal reflux disease such as reflux esophagitis, gastritis, duodenitis, gastric ulcer, duodenal ulcer, and peptic ulcer, it is recommended to use a proton pump inhibitor (PPI) as an early therapeutic agent. Proton pump inhibitor ingredients currently registered as therapeutic agents of, examples of proton pump inhibitors currently registered in Korea include esomeprazole, omeprazole, pantoprazole, lansoprazole, etc. In spite of successful early treatment, 75-92% of patients experience disease relapse. Particularly, it is reported that 80% of patients affected by gastroesophageal reflux disease (GERD) suffer nighttime heartburn, resulting in sleep disturbances or hampering daytime activities (Non-patent document 1).

In addition, proton pump inhibitors are known to be effective during the daytime when meals are consumed because they inhibit acid breakthrough by inactivating H+/K+ ATPase of parietal cells, and active acid secretion is essential for expression of drug action. Also, there is another limitation in that 24-hour sustained drug efficacy is lowered due to the short half-life of drug substances, which is usually about 2 hours. (Non-patent document 2).

In addition, nocturnal acid breakthrough (NAB) refers to a case where an intragastric pH is maintained to be less than 4 for greater than one hour during a nighttime (22:00-06:00), and the nocturnal acid breakthrough is recognized as one of the causes of recurrence of gastroesophageal reflux disease.

It is reported that gastroesophageal reflux disease patients experience symptoms such as heartburn or a burning sensation caused by the nocturnal gastroesophageal reflux disease, and discomfort due to gastric acid reflux of the esophagus, leading to a deterioration in their quality of life, and affecting their social life. However, conventional proton pump inhibitor formulations have a relatively short blood half-life, that is, only about two hours, and thus the efficacy of drug present in blood for 24 hours cannot be maintained. Therefore, only about 70% of proton pumps are suppressed, thus causing the occurrence of nocturnal gastroesophageal reflux diseases. In addition, the most influential factor in the digestive tract where orally administered drugs are directly absorbed is generally food intake, and esomeprazole as a proton pump inhibitor is also known to be a drug affected by diet and is inconvenient in drug-taking.

As such, there remain unfulfilled demands for improvements in nocturnal acid breakthrough, which could not be solved by conventional proton pump inhibitor formulations, and reducing dietary effects when taking medications. Accordingly, the present inventors completed the present disclosure by preparing an esomeprazole formulation having a dual release profile of immediate release and sustained release, identifying therapeutic effects of the formulation on nocturnal acid breakthrough, which could not be treated with conventional proton pump inhibitors, and developing a formulation capable of reducing dietary effects.

[Prior Art Literatures]

(Non-patent document 1) Nighttime Heartburn Is an Under-Appreciated Clinical Problem That Impacts Sleep and Daytime Function: The Results of a Gallup Survey Conducted on Behalf of the American Gastroenterological Association, Reza Shaker et al., THE AMERICAN JOURNAL OF GASTROENTEROLOGY, Vol. 98, No. 7, pp. 1487-1494

(Non-patent document 2) Mechanism of action and usage of Proton Pump Inhibitors, Journal of the Korean Society of Gastroenterology, 2006, vol. 48, pp. 4-8

DESCRIPTION OF EMBODIMENTS

TECHNICAL PROBLEM

Provided is a pharmaceutical composition comprising esomeprazole or a pharmaceutically acceptable salt thereof with a dual release profile.

Provided is also use for treating nocturnal acid breakthrough of the pharmaceutical composition.

## SOLUTION TO PROBLEM

An aspect of the present disclosure provides a pharmaceutical composition comprising an immediate release enteric-coated tablet comprising esomeprazole or a pharmaceutically acceptable salt thereof as an active ingredient and a sustained-release enteric-coated tablet comprising esomeprazole or a pharmaceutically acceptable salt thereof as an active ingredient, wherein

(i) the maximum concentration ($C_{max\ 0\text{-}3h}$) of the active ingredient in blood within 0 to 3 hours after administration is 40% to 80% of the maximum concentration ($C_{max\ 0\text{-}24h}$) in blood within 0 to 24 hours after administration,
(ii) a time ($T_{max}$) to reach the maximum blood concentration within 0 to 24 hours is 2 hours after administration; and
(iii) the $AUC_{3\text{-}24h}$ is at least 5 times $AUC_{0\text{-}3h}$.

The $AUC_{0\text{-}24h}$ of the composition may be 70 to 130% compared to $AUC_{0\text{-}24h}$ of the esomeprazole single-release formulation of the same dose.
The $AUC_{4\text{-}24h}$ of the composition may be 120 to 170% compared to $AUC_{4\text{-}24h}$ of the esomeprazole single-release formulation of the same dose.
The time taken for the composition to reach the maximum blood concentration ($C_{max\ 0\text{-}24h}$) within 0 to 24 hours may be 3 to 7 hours after administration.
The time ($T_{max}$) taken for the composition to reach the maximum blood concentration within 0 to 24 hours is 3 hours after administration.
The composition may be a capsule filled with a mixture of the immediate-release enteric-coated tablet and the sustained-release enteric-coated tablet.
The immediate-release enteric-coated tablet and the sustained-release enteric-coated tablet may be multi-unit spheroidal tablets (MUSTs).
The composition may include the immediate-release enteric-coated tablet and the sustained-release enteric-coated tablet in a ratio of 1:1.
Each of the immediate-release enteric-coated tablet and the sustained-release enteric-coated tablet of the pharmaceutical composition comprises a core comprising an active ingredient, and the core may further comprise one or more excipients selected from a diluent, a binder, a disintegrant, a lubricant, a surfactant, an antioxidant, a preservative, and a stabilizer.
The immediate-release enteric-coated tablet and sustained-release enteric-coated tablet include an inner coating layer formed on each core, and the inner coating layer may include one or more coating bases selected from the group consisting of hydroxypropyl methylcellulose (HPMC), polyvinylpyrrolidone (PVP), low-substituted hydroxypropyl cellulose (HPC-L), starch, gelatin, ethyl cellulose, and any combination thereof.
The immediate-release enteric-coated tablet may comprise an immediate-release enteric coating layer formed on the inner coating layer, and the sustained-release enteric-coated tablet may comprise a sustained-release enteric coating layer formed on the inner coating layer.
The composition may comprise esomeprazole or a pharmaceutically acceptable salt thereof as an active ingredient in an amount of 10 to 50 mg per unit dosage form.
The composition may be administered once a day.
The composition may be for use in preventing or treating nocturnal acid breakthrough.
The release characteristics of the composition may appear in a fasted condition or before having a meal when the composition is administered.

## ADVANTAGEOUS EFFECTS OF DISCLOSURE

The pharmaceutical composition with a dual release profile, comprising esomeprazole or a pharmaceutically acceptable salt thereof, according to the present disclosure, can secure bioavailability equivalent to that of the existing esomeprazole immediate-release enteric formulation, and, even when taken once a day, can prevent the occurrence of nocturnal acid breakthrough by maintaining the efficacy for a long time by a dual release profile and thus can be usefully used as a therapeutic agent for nocturnal acid breakthrough. In addition, the pharmaceutical composition according to the present disclosure can be taken regardless of the meal by reducing the deviation of drug absorption according to food intake (e.g., fasted or high-fat diet), thereby increasing the convenience in drug-taking.

## BRIEF DESCRIPTION OF DRAWINGS

FIG. 1 is a graph showing the dissolution rate (%) of esomeprazole according to the dissolution time (minutes) in formulations of Examples 1 to 3 and Comparative Example 1.

FIG. 2 is a graph showing the dissolution rate (%) of esomeprazole according to the dissolution time (minutes) in formulations of Example 4 and Comparative Example 2.

FIG. 3 is a graph showing the average concentration-time pattern of esomeprazole at each blood sampling time point after a single dose of formulations of Example 1 and Comparative Example 1, respectively.

FIG. 4 is a graph showing the average concentration-time pattern of esomeprazole at each blood sampling time point after a single dose of formulations of Example 4 and Comparative Example 2, respectively.

FIG. 5 shows the average concentration-time pattern of esomeprazole at each blood sampling time point after a single dose of a formulation of Example 1 according to dietary conditions (high-fat diet or fasted).

FIG. 6 shows graphs of integrated gastric acidity according to dietary conditions (high-fat diet or fasted) and time. In FIG. 6, BASE indicates a baseline, and DAY 1 indicates the graph after a single dose.

FIG. 7 shows a graph of average intragastric pH according to dietary conditions (high-fat diet or fasted) and time after a single dose of formulation of Example 1.

MODE OF DISCLOSURE

In an aspect, there is provided a pharmaceutical composition comprising esomeprazole or a pharmaceutically acceptable salt thereof and exhibiting a dual release profile. The pharmaceutical composition according to an aspect comprises

an immediate-release enteric-coated tablet comprising esomeprazole or a pharmaceutically acceptable salt thereof as an active ingredient and a sustained-release enteric-coated tablet comprising esomeprazole or a pharmaceutically acceptable salt thereof as an active ingredient, the pharmaceutical composition having the following release characteristics:

(i) the maximum blood concentration ($C_{max\ 0\text{-}3h}$) of the active ingredient within 0 to 3 hours after administration is 40% to 80% of the maximum blood concentration ($C_{max\ 0\text{-}24h}$) of the active ingredient within 0 to 24 hours after administration,

(ii) a time ($T_{max}$) to reach the maximum blood concentration within 0 to 24 hours is 2 hours after administration; and

(iii) $AUC_{3\text{-}24h}$ is at least 5 times $AUC_{0\text{-}3h}$.

The active ingredient of the pharmaceutical composition according to an aspect, the esomeprazole ((S)-5-methoxy-2-[(4-methoxy-3,5-dimethylpyridine-2-yl)methylsulphinyl]-3H-benzoimidazole) is a compound represented by Formula 1, known as an (S)-optical isomer of omeprazole of Formula 2.

[Formula 1]

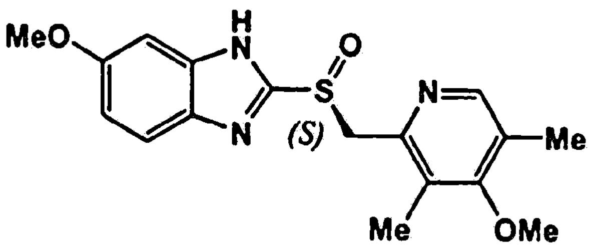

[Formula 2]

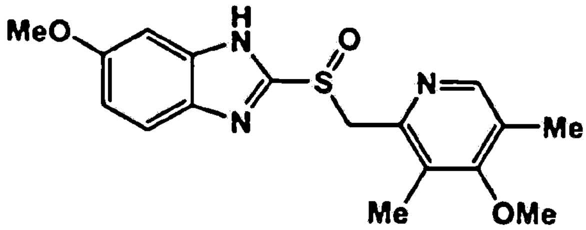

As used herein, the term "pharmaceutically acceptable salt" means a salt prepared by a general method in the related art, and the preparation method is publicly known to one skilled in the art. Specifically, the pharmaceutically acceptable salt may include, but not limited to, pharmaceutically or physiologically acceptable salts derived from the following inorganic acids or bases. The pharmaceutically acceptable salt of the esomeprazole according to the present disclosure may be a metal salt, such as a magnesium salt, a strontium salt, a lithium salt, a sodium salt, a potassium salt or a calcium salt, or an ammonium salt, but the pharmaceutically acceptable salt of esomeprazole is not limited thereto. More preferably, a magnesium salt of esomeprazole or a strontium salt of esomeprazole may be used as the pharmaceutically acceptable salt of esomeprazole. In one aspect, the esomeprazole or the pharmaceutically acceptable salt thereof may be used in the form of anhydride or hydrate.

As used herein, the term "area under the drug concentration-time response curve (AUC)", which is a graph representing the relationship between the administered drug concentration in blood and the time, refers to an area corresponding to a portion defined by the drawn curve and the horizontal axis, and is also referred to as an "area under the drug concentration curve" or an "area under the drug concentration-time curve". The term "$AUC_t$" refers to an area under the drug concentration-time curve from a drug administration time to a quantification time t of a final drug concentration in blood. The term "$AUC_{0-24h}$" refers to an area under the drug concentration-time curve over a 0-24 hour period after drug administration. The term "$AUC_{0-3h}$" refers to an area under the drug concentration-time curve over a 0-3 hour period after drug administration.

In the pharmaceutical composition according to an aspect, the dual release profile may be defined by limiting a corresponding feature of the area under the drug concentration-time curve to a time period in which the corresponding feature is exhibited, like $AUC_{0-24h}$. For example, in the present disclosure, the $AUC_{0-24h}$ indicates an area under the curve of drug concentration-time response of esomeprazole or a pharmaceutically acceptable salt thereof as an active ingredient, measured for a time period of first 24 hours after administration of the pharmaceutical composition, and the $AUC_{3-24h}$ indicates an area under the curve for a time period ranging from 3 hours to 24 hours after administration.

As used herein, the term "maximum blood concentration (maximum plasma concentration) ($C_{max}$)" refers to a maximum concentration of the drug in blood, measured after drug administration. The term "$C_{max\ 0-3h}$" refers to a maximum blood concentration measured within 3 hours after administration (0 hour). The term "$C_{max\ 0-24h}$" refers to a maximum blood concentration observed within 24 hours after drug administration (0 hour).

As used herein, the term "time ($T_{max}$) to reach the maximum blood concentration" refers to a time to reach the maximum blood concentration ($C_{max}$) after administration.

As used herein, the term "average" refers to an arithmetic mean, and when there are n data, it is a value obtained by dividing the sum of n data by n.

As used herein, the term "median value" means a middle value when the data are arranged according to size. However, if the number of data is even, there are two median values, so in this case, the average of these two values is taken as the median.

The dual-release pharmaceutical composition according to an aspect may exhibit any one or more of the following release characteristics (i) to (iii), or may simultaneously exhibit all of the following release characteristics (i) to (iii).

(i) The maximum blood concentration ($C_{max\ 0-3h}$) of the active ingredient in blood within 0 to 3 hours after administration may be 40% to 80% of the maximum blood concentration ($C_{max\ 0-24h}$) of the active ingredient within 0 to 24 hours after administration. For example, the $C_{max}$ of the active ingredient within 0 to 3 hours after administration may be 50 to 70% of the $C_{max}$ within 0 to 24 hours after administration.

(ii) The time ($T_{max}$) to reach the maximum blood concentration within 0 to 24 hours may be 2 hours after administration. For example, the time to reach the maximum concentration ($C_{max\ 0-24h}$) may be 2.1 hours, 2.2 hours, 2.25 hours, 2.5 hours, 3.0 hours, 3.1 hours, 3.5 hours, 4.0 hours, or 4.5 hours.

(iii) The $AUC_{3-24h}$ may be at least 5 times higher than the $AUC_{0-3h}$.For example, the $AUC_{3-24h}$ may be at least 5.5 times, at least 6.0 times, at least 6.5 times, at least 7.0 times, or at least 7.5 times higher than the $AUC_{0-3h}$.

Since the conventional proton pump inhibitors have a limitation in that the drug efficacy thereof may not be maintained for up to 24 hours due to their short half lives in blood, that is, at most about 2 hours.

However, since the dual release pharmaceutical composition of the present disclosure exhibits all of the release characteristics (i) to (iii), inclusive of an immediate release characteristic and a sustained release characteristic, the pharmaceutical composition of the present disclosure can maintain the drug efficacy thereof for an extended period of time, which is attributed to a dual release characteristic including both of an immediate release characteristic and a sustained release characteristic, and thus can increase a time period in which the intragastric pH is maintained to be 4.0 or higher.

In one aspect, the dual release pharmaceutical composition of the present disclosure has a 24-hour release characteristic equivalent to that of the conventional release formulation, that is not a dual-release formulation. That is to say, in one aspect, the dual release pharmaceutical composition of the present disclosure has an equivalent level of $AUC_{0-24h}$ as compared to that of an equal amount of a non-dual release esomeprazole formulation, for example, a single-release formulation, namely, a conventional immediate-release or enteric formulation comprising esomeprazole in an equal amount. In a specific embodiment, the equivalent level means that the extent of release equals to 70 to 130% of the $AUC_{0-24h}$ of a non-dual release formulation (a single-release formulation) relative to the $AUC_{0-24h}$ of the dual-release formulation. Here, the non-dual release esomeprazole formulation or the conventional immediate-release enteric esomeprazole formulation may mean a formulation comprising an enteric base therein and having an esomeprazole dissolution rate of less than 10% by weight for 120 minutes in 0.1 N HCl and an esomeprazole dissolution rate of 85% by weight for 30 minutes in an aqueous buffer solution having a pH 6.8, when a dissolution test is continuously conducted for 90 minutes at a temperature of 37±0.5° C at 100 revolutions per minutes (rpm) in the aqueous buffer solution of pH 6.8, following the dissolution in 300 mL of 0.1 N HCl aqueous buffer solution at 100 rpm for 120 minutes at a temperature of 37±0.5° C according to the paddle method II described in the United States Pharmacopeia (USP). Examples of the immediate release/enteric esomeprazole formulation include, but not limited to, an esomeprazole magnesium salt capsule marketed under the trade name of Nexium manufactured by AstraZeneca UK Limited.

In one aspect, the $AUC_{4-24h}$ of the pharmaceutical composition of the present disclosure may be greater than or equal to 120%, or less than or equal to 170%, preferably 120 to 170%, of that of the immediate-release enteric esomeprazole formulation of an equal amount. In another aspect, the pharmaceutical composition of the present disclosure may exhibit the time to reach the maximum blood concentration ($C_{max\ 0-24h}$) within 0 to 24 hours after administration is 3 to 7 hours after administration.

The $AUC_{4-24h}$ of the pharmaceutical composition of the present disclosure is defined as an area under drug concentration-time curve of esomeprazole as an active ingredient for a time period from 4 hours to 24 hours after administration.

The pharmaceutical composition of the present disclosure may include an active ingredient in separable forms of an immediate-release enteric-coated tablet and a sustained-release enteric-coated tablet in an appropriate proportion range. In an aspect, the pharmaceutical composition of the present disclosure may include as an active ingredient 5 to 100 mg, specifically 10 to 75 mg or 10 to 50 mg, more specifically about 20 mg, 40 mg or 50 mg of esomeprazole free base, or a pharmaceutically acceptable salt thereof in an equivalent amount as converted as an active ingredient, but not limited thereto. In the pharmaceutical composition, the immediate-release enteric-coated tablet and the sustained-release enteric-coated tablet may comprise esomeprazole or a pharmaceutically acceptable salt thereof at a weight ratio in a range of 2:1 to 1:2, for example, at a weight ratio of 1:1.

The dose of the pharmaceutical composition of the present disclosure may be in a range of, for example, about 0.001 mg/kg to about 100 mg/kg, about 0.01 mg/kg to about 10 mg/kg, or about 0.1 mg/kg to about 1 mg/kg, on an adult basis. The dose may be orally administered in a unit dosage form including once a day, multiple times a day, once a week, once every two weeks, once every three weeks, once every four weeks, or once a year, and the dose may be administered in the morning or in the afternoon.

The pharmaceutically effective amount or the effective dose of the pharmaceutical composition of the present disclosure may vary according to the preparation method, administration mode, administration time and/or administration route of the pharmaceutical composition, various factors such as kinds and extents of a therapeutic response to be achieved by administration of the pharmaceutical composition, the species, age, body weight, general health condition, symptoms or severity of disease, sex, diet, or rate of excretion of a subject being treated, or drugs or other ingredient used for the subject at the same time or at different times, and other factors well known in the medical field. One of ordinary skill in the art may readily determine and prescribe the effective dose for desired treatment.

In a specific embodiment, the pharmaceutical composition of the present disclosure reaches a time ($T_{max}$) in which the total weight of the active ingredient becomes a maximum blood concentration $C_{max}$ within about 3 to 7 hours when administered once a day in an amount equivalent to 40 mg of the esomeprazole free base, and exhibits a maximum concentration ($C_{max}$) of the esomeprazole free base in a range of 550 to 1,450 μg/L, and $AUG_{0-24h}$ of about 1,800 to 7,000 (see Table 9).

In another specific embodiment, the pharmaceutical composition of the present disclosure reaches a time ($T_{max}$)

in which the total weight of the active ingredient becomes a maximum blood concentration ($C_{max}$) within about 2.1 to 6 hours when it is administered once a day in an amount equivalent to 20 mg of the esomeprazole free base, and exhibits a maximum concentration ($C_{max}$) of the esomeprazole free base in a range of 250 to 750 μg/L, and $AUC_{0-24h}$ of about 900 to 3,500 (see Table 12).

The pharmaceutical composition of the present disclosure has a dual release profile, which is a characteristic part of the pharmaceutical composition, and can maintain drug efficacy for a long time owing to its, thereby preventing occurrence of gastroesophageal reflux disease. Conventional proton pump inhibitors have a limitation in that the drug efficacy thereof may not be maintained for up to 24 hours due to their short half lives in blood, that is, at most about 2 hours. However, the pharmaceutical composition of the present disclosure can effectively control nocturnal acid breakthrough by increasing a time period in which the intragastric pH is maintained to be 4.0 or higher. Therefore, the pharmaceutical composition of the present disclosure can be usefully used for treatment of nocturnal acid breakthrough.

As used herein, the term "nocturnal acid breakthrough (NAB)" refers to a nocturnal intragastric pH less than 4 for greater than one hour during a nighttime (between 10 p.m.- 6 a.m. of the next day). The nocturnal acid breakthrough may cause heartburn due to an abrupt pH drop in the stomach early in the morning. Since recurrence of gastric acid-related diseases may be induced and even a night's sleep may be disturbed, a solution to the nocturnal acid breakthrough has become a critical issue. However, currently available proton pump inhibitor (PPI) medication still has a limitation in overcoming NAB-related symptoms. The pharmaceutical composition of the present disclosure has treatment and amelioration effects of a general gastric acid-related disease as a proton pump inhibitor and can inhibit the nocturnal acid breakthrough accompanied by the general gastric acid-related disease. For example, when the composition of the present disclosure is administered once a day, percent of time periods in which the nocturnal intragastric pH of 4 or higher is maintained for greater than one hour during a nighttime period (between 10 p.m.- 6 a.m. of the next day) may be 45% or more for a single-dose administration and 65% or more for one week administration (see Table 10). For example, when the composition of the present disclosure is administered once a day, percent of time periods in which the nocturnal intragastric pH of 4 or higher is maintained for greater than one hour during a nighttime (between 10 p.m.- 6 a.m. of the next day) may be 35% or more for a single-dose administration and 60% or more for one week administration (see Table 13).

The pharmaceutical composition of the present disclosure may be a pharmaceutical composition used for prevention or treatment of nocturnal acid breakthrough. The term "prevention" means all actions of suppressing occurrence of the disease or delaying the onset thereof by administering the pharmaceutical composition. The term "treatment" refers to any action for improving or favorably changing symptoms of the disease by administration of the pharmaceutical composition.

The pharmaceutical composition of the present disclosure can also be used for prevention or treatment of a disease related to hypersecretion of gastric acid, selected from the group consisting of gastroesophageal reflux disease, gastritis, duodenitis, gastric ulcer, duodenal ulcer, and peptic ulcer.

In one aspect, the pharmaceutical composition of the present disclosure comprises: an immediate-release enteric-coated tablet comprising a core comprising esomeprazole or a pharmaceutically acceptable salt thereof as an active ingredient, an inner coating layer formed on the core, and an immediate-release enteric coating layer formed on the inner coating layer; and a sustained-release enteric-coated tablet comprising a core comprising esomeprazole or a pharmaceutically acceptable salt thereof as an active ingredient, an inner coating layer formed on the core, and a sustained-release enteric coating layer formed on the inner coating layer. In a specific embodiment, the esomeprazole is comprised in an amount of about 10 to 40% by weight, for example, 25 to 35% by weight, on the basis of a total weight of the core.

Since the esomeprazole is readily degraded or modified under acidic conditions, formulations including esomeprazole for oral administration need to be transmitted to a gastrointestinal tract where the pH level is almost neutral and the formulations can be rapidly absorbed. To this end, there is a need for a formulation capable of preventing the formulation from being exposed to the gastric acid in the stomach and including having an enteric coating layer for absorption in the intestines.

In one aspect, the pharmaceutical composition may be a composite capsule. The core including the esomeprazole or a pharmaceutically acceptable salt thereof may be any solid formulation that can be encapsulated in a capsule, and may be selected from the group consisting of, for example, a pellet, a mini tablet, a tablet, and a combination thereof. In a specific embodiment, the core may be in the form of a mini tablet, more specifically, in the form of a spheroidal mini tablet, and the immediate-release enteric-coated tablet and the sustained-release enteric-coated tablet, which are multi-unit spheroidal tablets (MUSTs), may be filled into the composite capsule. In one aspect, the core of the immediate-release enteric-coated tablet and the core of the sustained-release enteric-coated tablet may both be in forms of mini tablets, that is, MUSTs, and may be filled into the composite capsule.

The mini tablet may have a diameter in a range of 1 mm to 4 mm, more specifically in a range of 1.5 mm to 3 mm. The mini tablets may include at least 4 mini tablets, more specifically mini tablets defined by 4 to 40 independent layers, respectively, as immediate-release enteric-coated tablets and sustained-release enteric-coated tablets in the

capsule inner space. The mini tablet may be prepared by a general method known in the art. (Patent Document: KR2013-0115864)

The core may further include an arbitrary pharmaceutical additive that can be generally used in the art for the manufacture of the core in an appropriate amount, together with esomeprazole a pharmaceutically acceptable salt thereof as an active ingredient. For example, the core may further comprise one or more excipients selected from the group consisting of a diluent, a binder, a disintegrant, a lubricant, a surfactant, an antioxidant, an antiseptic, a stabilizer, and a combination thereof, but not limited thereto.

Suitable examples of the diluent may include, but not limited to, one or more selected from the group consisting of mannitol, microcrystalline cellulose, lactose, cellulose and a derivative thereof, dibasic or tribasic calcium phosphate, erythritol, low-substituted hydroxypropyl cellulose, pregelatinized starch, sorbitol, xylitol, and a combination thereof, and mannitol and/or microcrystalline cellulose may be used in a specific embodiment. In a specific embodiment, the diluent may be used in an amount of about 10% to about 50% by weight, for example, 25% to 45 % by weight, on the basis of the total weight of the core.

Suitable examples of the binder may include, but not limited to, one or more selected from the group consisting of hydroxypropyl cellulose (HPC), copovidone (copolymers of vinyl pyrrolidone with other vinyl derivatives), hydroxypropyl methylcellulose (HPMC), polyvinyl pyrrolidone (povidone), pregelatinized starch, low-substituted hydroxypropylcellulose (HPC-L), and a combination thereof, and hydroxypropyl cellulose may be used in a specific embodiment.

Suitable examples of the disintegrant may include, but not limited to, one or more selected from the group consisting of croscamellose sodium, corn starch, crospovidone, low-substituted hydroxypropylcellulose (HPC-L), pregelatinized starch, and a combination thereof, and croscamellose sodium may be used in a specific embodiment. In a specific embodiment, the disintegrant may be used in an amount of about 10% to about 30% by weight, for example, 15% to 35% by weight, on the basis of the total weight of the core.

Suitable examples of the lubricant may include, but not limited to, one or more selected from the group consisting of sodium stearyl fumarate, magnesium stearate, talc, polyethylene glycol, calcium behenate, calcium stearate, hydrogenated castor oil, and a combination thereof, and sodium stearyl fumarate sodium may be used in a specific embodiment. In a specific embodiment, the lubricant may be used in an amount of about 1% to about 10% by weight, for example, 2% to 8% by weight, on the basis of the core.

The inner coating layer formed on the core may include any hydrophilic polymer as a coating base, as long as it does not inhibit release of an active ingredient in the core during disintegration of the enteric coating layer upon administration of the composite capsule while blocking an interaction between the core and the enteric coating layer. Examples of the coating base of the inner coating layer may include one or more selected from the group consisting of hydroxypropyl methylcellulose (HPMC), polyvinylpyrrolidone (PVP), low-substituted hydroxypropyl cellulose (HPC-L), starch, gelatin, ethyl cellulose (EC), and a combination thereof. In a specific embodiment, the inner coating layer may include hydroxypropyl methylcellulose as a coating base.

In this specification, the core having the inner coating layer formed thereon is also referred to as an inner-coated core. The amount of the inner coating layer may be appropriately selected by one of ordinary skill in the art. However, in a specific embodiment, the inner coating layer may be comprised in an amount of about 2% to about 6% by weight, or about 3% to about 5% by weight, with respect to the inner-coated core.

In an aspect, an immediate-release enteric-coated tablet may be prepared by coating an immediate-release enteric coating layer on an inner-coated tablet having a core coated with the inner coating layer. In an aspect, a sustained-release enteric-coated tablet may be prepared by coating a sustained-release enteric coating layer on an inner-coated tablet having a core coated with the inner coating layer.

Since the composite capsule includes both of the immediate-release enteric-coated tablet and the sustained-release enteric-coated tablet, each including an enteric coating layer, acid resistance can be secured in the stomach being in a strongly acidic condition, upon reaching the intestines through the stomach, a first release of the active ingredient is rapidly done from the immediate-release enteric-coated tablet, and after a time of delay, a second release of the active ingredient is sequentially done from the sustained-release enteric-coated Therefore, upon reaching the intestines, the composite capsule can exhibit fast drug efficacy owing to rapid drug dissolution, while avoiding drug degradation in the stomach, and the drug efficacy can be sustained for a long time by the dual release of the drug.

The immediate-release enteric coating layer comprised in the immediate-release enteric-coated tablet may include as a coating base methacrylic acid copolymer LD. The methacrylic acid copolymer LD is an anionic copolymer including methacrylic acid and ethyl-acrylate at a ratio of about 1:1, and is present in the form of a solution. For example, the methacrylic acid copolymer LD may be a 20 to 40% dispersion marketed under the trade name of Eudragit L30 D-55. The IUPAC name of the methacrylic acid copolymer LD is poly(methacylic acid-co-ethyl acrylate) 1:1.

The sustained-release enteric coating layer comprised in the sustained-release enteric-coated tablet may comprise as a coating base a mixture of methacrylic acid copolymer S and methacrylic acid copolymer L mixed at a ratio of 1.5:1 to 3.5:1 (w/w), more specifically 2:1 to 3:1 (w/w).

The methacrylic acid copolymer S, an anionic copolymer including methacrylic acid and methyl methacrylate

at a ratio of about 1:2, is marketed under the trade name of Eudragit S-100, and the IUPAC name thereof is poly(methacylic acid-co-methyl methacrylate) 1:2.

The methacrylic acid copolymer L, an anionic copolymer including methacrylic acid and methyl methacrylate at a ratio of about 1:1, is marketed under the trade name of Eudragit L-100. The IUPAC name of the methacrylic acid copolymer L is poly(methacylic acid-co-ethyl acrylate) 1:1. The methacrylic acid copolymer LD, which is a coating base of the sustained-release enteric coating layer, may be comprised in an amount of about 5% to about 50% (w/w), more specifically, about 8% to about 30% (w/w), as a solid content with respect to in the inner-coated core. When the solid content of the coating base is less than 5% (w/w) with respect to in the inner-coated core, it is difficult to secure acid resistance in the 0.1 N HCl aqueous solution, in spite of rapid dissolution. Hence, when orally administered, the active ingredient may be degraded due to the nature of the PPIs, and accordingly, the drug efficacy is hardly exhibited. In addition, when the solid content of the coating base is greater than 50% (w/w) with respect to the inner-coated core, sufficient acid resistance may be secured in the 0.1 N HCl aqueous solution, but after passing through the stomach, the dissolution of the active ingredient is excessively slowed in the intestines, resulting in a delay in the in vivo drug absorption and a delayed drug efficacy.

The methacrylic acid copolymer S and the methacrylic acid copolymer L, which are the coating bases of the sustained-release coating layer may be mixed at a mixing ratio of about 1.5:1 (w/w) to about 3.5:1 (w/w), and for example, about 2:1 (w/w) to about 3:1 (w/w). When the mixing ratio of the coating bases is lower than the ratio stated above, a proportion of the methacrylic acid copolymer L, which is dissolved at a relatively low pH, becomes relatively high, and accordingly, rapid release may occur upon reaching the intestines through the stomach, and due to the rapid dissolution upon reaching the intestines, the dual release characteristic may be hardly exhibited. In addition, when the mixing ratio of the coating bases is higher than the ratio stated above, a proportion of the methacrylic acid copolymer S, which is a relatively insoluble substance, is increased, resulting in excessively delayed release of the drug. Hence, the coating bases may be excreted without achieving complete releasing of the drugs, resulting in low bioavailability.

The mixture of the methacrylic acid copolymer S and the methacrylic acid copolymer L may be comprised in an amount of about 5% to about 40% (w/w) as a solid content, for example, about 15% to about 35% (w/w), as a solid content with respect to the inner-coated core. When the solid content of the mixture is less than the content range stated above, a desired dissolution delay of the sustained-release enteric-coated tablet cannot be secured, and due to the rapid dissolution upon reaching the intestines, the dual release characteristics may be hardly exhibited. In addition, when the solid content of the mixture is greater than the content range stated above, the enteric coating layer may become thicker so that the release of the sustained-release enteric-coated tablet may be excessively delayed. Thus, the drug in the composite capsule may be excreted without achieving complete releasing of the drug, resulting in low bioavailability.

The capsule constituting the composite capsule may be a hard capsule, and any hard capsule generally used in the art may be used as the hard capsule. A base of the hard capsule may be, for example, selected from gelatin, hypromellose (hydroxypropylmethylcellulose (HPMC)), pullulan (NP caps TM, etc., Capsugel Company), polyvinyl alcohol, and a combination thereof, but not limited thereto.

In the composite capsule, the active ingredient is hardly released in the strongly acidic environment. However, the first release of the active ingredient rapidly proceeds from the immediate-release enteric-coated tablet in the intestines at a pH of 5.5 or less, and the second release of the active ingredient proceeds from the sustained-release enteric-coated tablet in the intestines at a pH of 6.5 to 7.0.

During a dissolution test, the composite capsule is left for 2 hours in 0.1 N HCl aqueous solution, and the resulting solution is then transferred an artificial intestinal fluid at about 6.8 pH. The release hardly occurs during the first two hours, but the release starts in the artificial intestinal fluid. At 135 minutes following the start of the dissolution test, about 35% (w/w) to about 65% (w/w) of the active ingredient of the composite capsule is released, and at 210 minutes after the start of the dissolution test, 75% or more (w/w) is released.

When a dissolution test is continuously conducted at 100 rpm for 90 minutes in an artificial intestinal fluid having a pH of 6.8 at a temperature of 37±0.5°C, following the dissolution in 0.1 N HCl aqueous solution, 300 mL at 100 rpm for 120 minutes at a temperature of 37±0.5°C according to the paddle method II described in the United States Pharmacopeia (USP), the composite capsule has acid resistance in 0.1 N HCl for 120 minutes, about 40%(w/w) to 60%(w/w) of the active ingredient is dissolved in the artificial intestinal fluid for 15 minutes, and about 75%(w/w) or more of the active ingredient is dissolved in the artificial intestinal fluid for 90 minutes the 0.1N HCl.

When the cores of the immediate-release enteric-coated tablet and the sustained-release enteric-coated tablet of the composite capsule are mini tablets (MUSTs) or tablets, the cores may be produced by direct compression or indirect compression, and dry granules or wet granules may be used in the indirect compression.

In a specific embodiment, a method of preparing the core may include (a) mixing esomeprazole or a pharmaceutically acceptable salt thereof with a diluent; (b) adding a disintegrant, binder and a lubricant to the mixture of the step (a) and mixing the resulting product; (c) subjecting the mixture of the step (b) to dry granulation, followed by tableting the resulting product to obtain a mini-tablet or a tablet.

Any coating method may be applied to the coating of the mini-tablet or the tablet with the inner coating layer,

the immediate-release enteric coating layer, and the sustained-release enteric coating layer. For example, a fluidized-bed coater may be used for the coating.

In one aspect, the immediate-release enteric coating layer of the pharmaceutical composition may include, as a coating base, methacrylic acid copolymer LD, in an amount of about 5% to about 50% (w/w) with respect to the core having the inner coating layer, and the sustained-release enteric coating layer of the pharmaceutical composition may include, as a coating base, a mixture of methacrylic acid copolymer S and methacrylic acid copolymer L in a mixing ratio of about 1.5:1 to about 3.5:1 (w/w) in an amount of about 10% to about 30% (w/w).

The pharmaceutical composition of the present disclosure may be prepared by a preparation method including the following steps: preparing a core including esomeprazole or a pharmaceutically acceptable salt thereof and an additive; coating the core with an inner coating layer; acquiring an immediate-release enteric-coated tablet by coating an immediate-release enteric coating layer on the inner coating layer; acquiring a sustained-release enteric-coated tablet by separately coating a sustained-release enteric coating layer on the inner coating layer; filling a capsule with the immediate-release enteric-coated tablet and the sustained-release enteric-coated tablet together to thus prepare a composite capsule.

The present disclosure relates to a method for preventing or treating nocturnal acid breakthrough, comprising a step of administering the pharmaceutical composition according to the present disclosure in a therapeutically effective amount to mammals including humans, the pharmaceutical composition comprising an immediate-release enteric-coated tablet and a sustained-release enteric-coated tablet, each including esomeprazole or a pharmaceutically acceptable salt thereof as the active ingredient according to the present disclosure.

The present disclosure relates to a method for prevention or treatment of a disease related to hypersecretion of gastric acid, including gastroesophageal reflux disease such as reflux esophagitis, gastritis, duodenitis, gastric ulcer, duodenal ulcer, and peptic ulcer, the method comprising a step of administering to mammals including humans the pharmaceutical composition in a therapeutically effective amount, the pharmaceutical composition comprising an immediate-release enteric-coated tablet and a sustained-release enteric-coated tablet, each including esomeprazole or a pharmaceutically acceptable salt thereof as the active ingredient according to the present disclosure.

As used herein, the term "subject" means mammals including monkeys, cows, horses, dogs, cats, rabbits, rats or mice. The prevention or treatment method includes administration of a therapeutically effective amount.

As used herein, the term "therapeutically effective amount" represents an amount of a pharmaceutical composition comprising an immediate-release enteric-coated tablet and a sustained-release enteric-coated tablet, each including esomeprazole or a pharmaceutically acceptable salt thereof as an active ingredient, which is effective to treat diseases related to hypersecretion of gastric acid, including gastroesophageal reflux disease such as reflux esophagitis, gastritis, duodenitis, gastric ulcer, duodenal ulcer, and peptic ulcer.

The prevention or treatment method of the present disclosure may comprise, by administering the pharmaceutical composition according to the present disclosure, not only handling a disease itself even before manifestation of a symptom thereof but also inhibiting or avoiding the symptom of the disease. In controlling a disease, the preventive or therapeutic amount of a particular active ingredient will vary according to the nature and severity of the disease or condition and the administration route of the active ingredient. The dose or frequency of administration will vary according to the age, weight or response of an individual subject. Suitable dosage regimens can be easily selected by one of ordinary skill in the art consistently with considerations of these factors. In addition, the prevention or treatment method of the present disclosure may further comprise administering a therapeutically effective amount of an additional active agent helpful for the disease treatment together with the pharmaceutical composition according to the present disclosure. The additional active agent may exhibit a synergistic or additive effect with the pharmaceutical composition of the present disclosure.

The prevention or treatment method of the present disclosure may comprise administering the pharmaceutical composition of the present disclosure at a dose in a range of, for example, about 0.001 mg/kg to about 100 mg/kg, about 0.01 mg/kg to about 10 mg/kg, or about 0.1 mg/kg to about 1 mg/kg, on an adult basis. The dose may be orally administered in a unit dosage form including once a day, multiple times a day, once a week, once every two weeks, once every three weeks, once every four weeks, or once a year, and the dose may be administered in the morning or in the afternoon.

The present disclosure provides a use of a pharmaceutical composition for prevention or treatment of nocturnal acid breakthrough, the pharmaceutical composition comprising an immediate-release enteric-coated tablet comprising esomeprazole or a pharmaceutically acceptable salt thereof as an active ingredient and a sustained-release enteric-coated tablet comprising esomeprazole or a pharmaceutically acceptable salt thereof as the active ingredient according to the present disclosure.

The present disclosure provides a use of a pharmaceutical composition for prevention or treatment of a disease related to hypersecretion of gastric acid, including gastroesophageal reflux disease such as reflux esophagitis, gastritis, duodenitis, gastric ulcer, duodenal ulcer, and peptic ulcer, the pharmaceutical composition comprising an immediate-release enteric-coated tablet and a sustained-release enteric-coated tablet, each including esomeprazole or a pharmaceutically acceptable salt thereof as the active ingredient according to the present disclosure.

The present disclosure provides a use of a pharmaceutical composition for preparing a drug for prevention or treatment of nocturnal acid breakthrough, the pharmaceutical composition comprising an immediate-release enteric-coated tablet and a sustained-release enteric-coated tablet, each including esomeprazole or a pharmaceutically acceptable salt thereof as the active ingredient according to the present disclosure.

The present disclosure provides a use of a pharmaceutical composition for preparing a drug for prevention or treatment of a disease related to hypersecretion of gastric acid, including gastroesophageal reflux disease such as reflux esophagitis, gastritis, duodenitis, gastric ulcer, duodenal ulcer, and peptic ulcer, the pharmaceutical composition comprising an immediate-release enteric-coated tablet and a sustained-release enteric-coated tablet, each including esomeprazole or a pharmaceutically acceptable salt thereof as the active ingredient according to the present disclosure.

The pharmaceutical composition of the present disclosure for preparing a drug may be mixed with a pharmaceutically acceptable carrier, and may further comprise other agonists.

The release characteristics of the pharmaceutical composition of the present disclosure may be exhibited in the fasted condition or prior to a meal, following the administration of the pharmaceutical composition.

The pharmaceutical composition of the present disclosure may be administered at a dose of 20 mg or 40 mg of esomeprazole, as necessary, once a day or twice a day, the administration may be performed over a period of a week, two weeks, four weeks or eight weeks according to the desired treatment purpose, and the dose may be appropriately increased or decreased according to the symptoms of the disease.

When the composition according to one embodiment is administered once a day, the ratio in which the pH in the stomach is maintained at 4.0 or higher for 24 hours after drug administration can be increased compared to before drug administration. The ratio in which the pH in the stomach is maintained at 4.0 or higher at night, for example, 12 hours to 22 hours, 13 hours to 22 hours, or 16 hours to 20 hours after drug administration can be increased by administering the composition according to one embodiment.

As used herein, the term "investigational products" refers to a test drug and a control drug.

The details discussed above with regard to the composition of the present disclosure, treatment methods and uses can be applied in the same manner unless otherwise clearly contradicted by context.

Numerical values set forth herein are intended to include the meaning of "about" even if not specified. As used herein, the term "about" means that the referenced value may vary to some extent, for example, 10%, 5%, 2%, or 1%.

As used herein, the terms "have", "may have", "comprise", or "may comprise" indicate the presence of a corresponding feature (e.g., a numerical value or a component such as an ingredient). and does not exclude the presence of additional features.

Hereinafter, the present invention will be described in detail with reference to Examples below. However, Examples below are illustrative examples of embodiments and are not intended to otherwise limit the scope of embodiments in any way.

Example 1: (1) Preparation of dual-release tablet comprising magnesium salt of esomeprazole

Using the compositions listed in Table 1 below, a magnesium salt of esomeprazole and mannitol were mixed, and the mixture was sieved using a 30 mesh round sieve. The prepared mixture was added to an empty blender with low-substituted hydroxypropyl cellulose, croscarmellose sodium, hydroxypropyl cellulose, and sodium stearyl fumarate to be blended for 15 minutes, thereby preparing a final mixture. The final mixture was put into a roller compactor, and then, subjected to dry granulation. Granules obtained therefrom were sieved using a 20 mesh round sieve.

Next, the resulting granules were punched using a multi-unit spheroidal tablet (MUST) punch having a diameter of 2.0 mm to yield 10 mini-tablets each having a hardness of about 1 to 2 kp and a weight of 75 mg. Next, the tablets were coated according to the compositions of Table 1 using a fluidized-bed coater, thereby obtaining tablets each having a coated inner layer (inner-coated tablets).

[Table 1]

| Process | Ingredient | Weight (mg/10 tablets) |
|---|---|---|
| Core | Esomeprazole magnesium trihydrate | 22.3 |
| | Mannitol | 28.7 |
| | Low-substituted hydroxypropylcellulose | 13.8 |
| | Croscarmellose sodium | 4.8 |
| | Hydroxypropylcellulose | 2.4 |
| | Sodium stearyl fumarate | 3.0 |

(continued)

| Process | Ingredient | Weight (mg/10 tablets) |
|---|---|---|
| **Total weight of core** | | **75.0** |
| Inner coating layer | Hypromellose 2910 | 3.8 |
| | Talc | 0.1 |
| | Purified water | (34.0) |
| | Sum of inner coating layers | 3.9 |
| **Total weight of inner-coated tablet** | | **78.9** |

Immediate-release enteric-coated tablets were obtained by enterically coating the inner-coated tablets by using the compositions of Table 2.

[Table 2]

| Process | Ingredient | Weight (mg/10 tablets) |
|---|---|---|
| **Total weight of inner-coated tablet** | | **78.9** |
| Enteric coating layer (Immediate-release enteric coating layer) | Methacrylic acid·ethyl acrylate copolymer (1:1) 30% dispersion | 22.32 (6.7 as solid content) |
| | Ethyl citrate | 1.5 |
| | Glycerin monostearate | 0.36 |
| | Polysorbate 80 | 0.14 |
| | Purified water | (14.8) |
| | Sum of enteric coating layers | 8.7 |
| **Total weight of im mediate-release enteric-coated tablet** | | **87.6** |

By using the prepared inner-coated tablets, the sustained-release enteric-coated tablets were enterically coated according to the compositions of Table 3 to obtain sustained-release enteric-coated tablets.

[Table 3]

| Process | Ingredient | Weight (mg/10 tablets) |
|---|---|---|
| **Total weig ht of inner-coated tablet** | | **78.9** |
| Sustained-release enteric coating layer | Methacrylic acid·methyl methacrylate copolymer (1:2) | 8.24 |
| | Methacrylic acid methyl methacrylate copolymer (1:1) | 4.12 |
| | Ethyl citrate | 1.22 |
| | Talc | 6.18 |
| | Red iron oxide | 0.04 |
| | Purified water | (17.4) |
| | Ethanol | (158.8) |
| | Sum of sustained-release coating layers | 19.8 |
| **Total weight of s ustained-release enteric-coated tablet** | | **98.7** |

The enteric-coated tablets (immediate-release enteric-coated tablets) and the sustained-release coated tablets (sustained-release enteric-coated tablets), obtained above, were lubricated using talc of 2.0 mg/T, respectively, and then 10 enteric-coated tablets and 10 sustained-release enteric-coated tablets were then filled into a hard capsule #2.

Example 2 to 3: Preparation of dual-release tablets comprising magnesium salt of esomeprazole

While the proportions of the compositions of the sustained-release coating layers listed in Table 3 were maintained in the sustained-release enteric-coated tablets of Example 1, the weights of the sustained-release coating layers were reduced to about 16.5 mg in Example 2 (95.4 mg in the total weight of sustained-release enteric-coated tablets), and were increased to about 26.0 mg in Example 3 (104.9 mg in the total weight of sustained-release enteric-coated tablets), respectively.

In detail, the composition of the sustained-release coating layer of Example 2 are shown in Table 4.

[Table 4]

| Process | Ingredient | Weight (mg/10 tablets) |
|---|---|---|
| **Total wei ght of inner-coated tablet** | | **78.9** |
| Sustained-release enteric coating layer | Methacrylic acid methyl methacrylate copolymer (1:2) | 6.87 |
| | Methacrylic acid methyl methacrylate copolymer (1:1) | 3.43 |
| | Ethyl citrate | 1.02 |
| | Talc | 5.15 |
| | Red iron oxide | 0.03 |
| | Purified water | (14.5) |
| | Ethanol | (132.3) |
| | Sum of sustained-release coating layers | 16.5 |
| **Total weight of s ustained-release enteric-coated tablet** | | **95.4** |

The composition of the sustained-release coating layer of Example 3 are shown in Table 5.

[Table 5]

| Process | Ingredient | Weight (mg/10 tablets) |
|---|---|---|
| **Total weight of inner-coated tablet** | | **78.9** |
| Sustained-release enteric coating layer | Methacrylic acid methyl methacrylate copolymer (1:2) | 10.82 |
| | Methacrylic acid methyl methacrylate copolymer (1:1) | 5.41 |
| | Ethyl citrate | 1.60 |
| | Talc | 8.12 |
| | Red iron oxide | 0.05 |
| | Purified water | (22.8) |
| | Ethanol | (208.5) |
| | Sum of sustained-release coating layers | 26.0 |
| **Total weight of i** | **mmediate-release enteric-coated tablet** | **104.9** |

Example 4: (2) Preparation of dual-release tablet comprising magnesium salt of esomeprazole

The enteric-coated tablets (immediate-release enteric-coated tablets) and the sustained-release coated tablets (sustained-release enteric-coated tablets), obtained in Example 1, were lubricated using talc of 2.0 mg/T, respectively, and then 5 enteric-coated tablets and 5 sustained-release enteric-coated tablets were then filled into a hard capsule #2.

Comparative Example 1: Preparation of immediate-release enteric formulation comprising magnesium salt of esomeprazole

In Comparative Example 1, 40 mg of Nexium®, which is commercially available in the market, was used as the immediate-release enteric formulation comprising magnesium salt of esomeprazole.

Comparative Example 2: Preparation of immediate-release enteric formulation comprising magnesium salt of esomeprazole

In Comparative Example 2, 20 mg of Nexium®, which is commercially available in the market, was used as the immediate-release enteric formulation comprising magnesium salt of esomeprazole.

Test Example 1. Dissolution Test

Using the formulations of Examples 1 to 4 and Comparative Examples 1 and 2, dissolution rates of esomeprazole were measured under the following conditions.

< Dissolution Conditions>

Eluate: Fluid having pH 6.8 changed by adding 700 mL of 0.086 M $NaHPO_4$ following a 2 hour test in 300 mL of 0.1 N HCl under acid resistance conditions Apparatus: USP paddle method, 100 ±2 rpm

Temperature: 37 ±0.5 °C

Time in which dissolution rates were measured: 125 minutes, 130 minutes, 135 minutes, 150 minutes, 180 minutes, and 210 minutes

<Analysis Conditions>

Device in use: HPLC (Hitachi 5000 series, Japan)
Detector: Ultraviolet spectrophotometer (measured wavelength: 302 nm)
Column: Stainless steel tube having an inner diameter of about 4.6 mm and a length of about 15 cm
5 μm column filled with silica gel for liquid chromatography
Mobile Phase: Sodium phosphate buffer (pH 7.3):acetonitrile:purified water=50:35:15
Flow rate: 1.0 mL/minute
Column temperature: 30° C

The measured dissolution rates of esomeprazole are shown in Tables 6 and 7 and FIGs. 1 and 2.

[Table 6]

| | | | Esomep razole dissolution rate (%) | | | | | |
|---|---|---|---|---|---|---|---|---|
| | Example 1 | | Example 2 | | Example 3 | | Comparative Example 1 | |
| Time (min) | Mean | SD | Mean | SD | Mean | SD | Mean | SD |
| 120 | 0.0 | 0.0 | 0.0 | 0.0 | 0.0 | 0.0 | 0.0 | 0.0 |
| 125 | 36.8 | 3.0 | 37.2 | 3.2 | 38.2 | 4.0 | 4.8 | 1.5 |
| 130 | 48.0 | 4.8 | 46.2 | 3.9 | 45.8 | 4.2 | 39.9 | 4.2 |

(continued)

| | Esomep razole dissolution rate (%) | | | | | | | |
|---|---|---|---|---|---|---|---|---|
| | Example 1 | | Example 2 | | Example 3 | | Comparative Example 1 | |
| Time (min) | Mean | SD | Mean | SD | Mean | SD | Mean | SD |
| 135 | 47.5 | 2.4 | 47.6 | 6.1 | 46.8 | 4.5 | 77.0 | 3.7 |
| 150 | 61.7 | 4.6 | 77.2 | 6.5 | 49.1 | 4.5 | 101.5 | 1.8 |
| 165 | 79.5 | 3.0 | 91.6 | 5.6 | 63.6 | 7.3 | 97.1 | 1.5 |
| 180 | 82.5 | 3.1 | 92.8 | 4.8 | 78.9 | 4.5 | 94.1 | 1.3 |
| 210 | 86.2 | 2.9 | 91.7 | 4.6 | 80.8 | 4.7 | 88.7 | 1.3 |

[Table 7]

| | Esomeprazole dissolution rate (%) | | | |
|---|---|---|---|---|
| | Example 4 | | Comparative Example 2 | |
| Time (min) | Mean | SD | Mean | SD |
| 120 | 0.0 | 0.0 | 0.0 | 0.0 |
| 125 | 46.1 | 1.8 | 13.0 | 2.7 |
| 130 | 49.8 | 1.1 | 70.8 | 4.6 |
| 135 | 49.9 | 1.9 | 93.5 | 3.9 |
| 150 | 59.8 | 5.0 | 99.0 | 1.6 |
| 165 | 81.3 | 2.1 | 100.1 | 1.4 |
| 180 | 84.4 | 2.3 | 98.2 | 1.2 |
| 210 | 87.2 | 2.4 | 92.7 | 1.2 |

Compared to Comparative Example 1 in which a single release profile was exhibited, Examples 1 to 3 distinctly exhibited a dual release profile of esomeprazole as an active ingredient. In Examples 1 to 3, 10 enteric-coated tablets (immediate-release enteric-coated tablets) and 10 sustained-release coating tablets (sustained-release enteric-coated tablets) were filled into one capsule. After two hour dissolution under acid resistance conditions, the 10 enteric-coated tablets were rapidly dissolved when the pH level of the eluate was changed to 6.8, confirming that most of the drug ingredient comprised in the enteric-coated tablets was released at 135 minutes.

Thereafter, as the sustained-release coating tablets are dissolved, the dual release profile was confirmed with dissolution of 75% or more at 135 to 180 minutes. When the dissolution profile of Example 1 was compared with the dissolution profiles of Examples 2 and 3, it was confirmed that similarity factors ($f_2$) were all 50 greater, suggesting that the dissolution profiles are similar.

Hereinafter, testing was conducted in two tests: Part A (40 mg); and Part B (20 mg), according to the investigational product dose groups.

Test Example 2. In vivo Pharmacokinetic Study: PK profile (1), Part A (40 mg)

To compare pharmacokinetic (PK) and pharmacodynamic (PD) properties of oral administration in healthy adult male volunteer subjects after oral administration, randomized, open-label, multiple dose and crossover studies were conducted by using each 40 mg of the tablet of Example 1 and Nexium® of Comparative Example 1 as investigational products.

A total of 48 volunteers selected as test subjects were randomized and divided into two sequence groups, and two-period crossover studies were then conducted. The investigational products corresponding to the respective sequence groups were administered to the test subjects in multiple doses once a day for a total of 7 days.

All test subjects were admitted to the clinical laboratory in the afternoon two days prior to a first dose of the

investigational products for each period and randomized according to the sequence groups.

[Table 8]

| Part A: 40 mg dose group (N=48) | | | |
|---|---|---|---|
| Se quence group | Number of test subjects | Period 1 | Period 2 |
| 1 | 24 | Comparative Example 1 | Example 1 |
| 2 | 24 | Example 1 | Comparative Example 1 |

A 7-day washout period was given between Period 1 and Period 2, and investigational products of Comparative Example 1 or Example 1 were administered to all of the test subjects with 150 mL water at the same time point of the morning hours.

One day prior to a first dose, the test subjects were subjected to 24-hour pH monitoring in the fasted condition for baseline evaluation at given time points. The test subjects were administered the tablet of Example 1 or Comparative Example 1 in the fasted condition according to the groups assigned to the first day administration group of investigational products, followed by conducting 24-hour pH monitoring and pharmacokinetic blood collections according to the predefined schedule. For the next 5 days, the test subjects were subjected to PK blood collection prior to administration, and then administered the investigational products at given time points according to the groups assigned thereto. The investigational products were administered to the test subjects in the fasted condition according to the groups assigned on the 7th day of administration, followed by conducting 24-hour pH monitoring and PK blood collections according to the predefined schedules.

After administration of the tablet of Example 1 or Comparative Example 1, blood collections planed for PK evaluation were all completed, and PK analysis was then conducted on 44 subjects with quantifiable drug concentrations.

After a single dose of each of the tablets of Example 1 and Comparative Example 1, average concentration-time profiles of esomeprazole were evaluated for each blood collection time, and the results thereof are presented in FIG. 2. Comparative Example 1, which is for commercially available formulations, exhibited a maximum blood concentration ($C_{max}$) level at about 2 hours after drug administration. Example 1 exhibited a maximum blood concentration ($C_{max}$) level at about 4 hours after administration. Whereas Comparative Example 1 exhibited a single peak indicating a single release profile of esomeprazole, Example 1 exhibited double peaks indicating a dual release profile of esomeprazole. A first peak appeared about 1.75 hours after drug administration, and a second peak of the double peaks appeared about 4.5 hours after drug administration.

PK parameters obtained after a single dose of each of the tablets of Example 1 and Comparative Example 1 are presented in Table 9 for comparison.

Table 9]

| Parameter | Example 1 (n=44) | | | Comparative Example 1 (n=44) | | |
|---|---|---|---|---|---|---|
| | Mean | SD | Variance coefficient (%) | Mean | SD | Variance coefficient (%) |
| $C_{max}$ (μg/L) | 1007.55 | 421.56 | 41.84 | 1622.18 | 544.04 | 33.54 |
| $AUC_{0-24h}$ (h × μg/L) | 4258.11 | 2464.43 | 57.89 | 4460.37 | 2461.37 | 55.19 |
| $T_{max}$ (h)$^{1)}$ | 4.00 [0.98 - 5.50] | | | 2.00 [0.73 - 4.52] | | |
| $t_{1/2}$ (h) | 1.60 | 0.35 | 21.51 | 1.54 | 0.50 | I 32.55 |

In the dose groups of the tablets of Example 1 and Comparative Example 1, the arithmetic mean $AUC_{0-24h}$ values were 4258.11 h×μg/L and 4460.37 h×μg/L, and the $C_{max}$ values were 1007.55 μg/L and 1622.18 μg/L, respectively.

In addition, compared to the Comparative Example 1-administered group, the $AUC_{0-24h}$ ($AUC_{last}$) geometric mean ratio and 90% confidence interval of the Example 1-administered group were 0.9479 and [0.9056 - 0.9922], respectively, and were included in Log 0.80 - Log 1.25, the equivalence criterion defined in the statistical analysis protocol.

After a single dose of the tablet of Example 1, the $C_{max}$ values were 537.1 μg/L and 884.7 μg/L at 0 to 3 hours and at 0 to 24 hours respectively, while, after a single dose of the tablet of Comparative Example 1, the $C_{max}$ values were 976.9 μg/L and 1167.1 μg/L, respectively.

After a single dose of the tablet of Example 1, the $AUC_{0-3h}$ and $AUC_{3-24h}$ values were 92.8 h×μg/L and 709.0 h×μg/L at 0 to 3 hours and at 3 to 24 hours, respectively. However, after a single dose of the tablet of Comparative

Example 1, the $AUC_{0-3h}$ and $AUC_{3-24h}$ values were 275.7 h×μg/L and 493.6 h×μg/L at 0 to 3 hours and at 0 to 24 hours, respectively.

After a single dose of the tablet of Example 1, the $AUC_{4-24h}$ value was 628.5 h×μg/L, while, after a single dose of the tablet of Comparative Example 1, the $AUC_{4-24h}$ value was 435.1 h×μg/L.

The $T_{max}$ (h)[1)] means the time to reach the maximum blood concentration and is expressed as the median value. After a single dose of the tablet of Example 1, the $T_{max}$ (h)[1)] value was 4.00, while, after a single dose of the tablet of Comparative Example 1, the $T_{max}$ (h)[1)] was 2.00.

To develop a formulation capable of maintaining drug efficacy for a long time, a method for delaying the release of a drug is generally used. If a drug release is excessively delayed, complete drug release may not be achieved until the formulation passes through the absorption site in the digestive tract. In this case, compared the conventional immediate release, the overall quantity of the drug absorbed may be reduced, as confirmed by AUC. The reduced AUC due to excessively delayed release means a reduction in the bioavailability, lowering the drug efficacy. In Example 1, the drug concentration in blood was maintained longer than in Comparative Example 1 in which the commercially available Nexium® tablet was used, while maintaining an equivalent level of AUC. Therefore, it was confirmed that the drug efficacy of Example 1 was maintained for a long time without lowering the bioavailability, compared to that of Comparative Example 1.

Test Example 3. In vivo Pharmacodynamics Study: Therapeutic effect of NAB (1), Part A (40 mg)

After the dose of the tablet of Example 1 or Comparative Example 1 as an investigational product, 24-hour pH monitoring was all completed, pharmacodynamic (PD) analysis was performed on 41 subjects who offered evaluable intragastric pH data. Drug administration was started from 9 am, and intragastric pH was measured for 24 hours.

With respect to the intragastric pH, when each of the tablets of Example 1 and Comparative Example 1 was administered in a single dose and multiple doses of 40 mg once a day for 7 days (40 mg dose of esomeprazole), the "percent of time periods in which the intragastric pH is greater than or equal to 4.0" and the "percent of the number of subjects having experienced NAB" are presented in Table 10.

[Table 10]

| Parameter | Period | Example 1 (n=41) | Comparative Example 1 (n=41) |
|---|---|---|---|
| | | Mean (SD) | Mean (SD) |
| Percent (%) of time periods in which pH≥4 for 24 hours | Baseline | 6.75(6.35) | 8.49(7.50) |
| | After the 1st dose | 56.22(15.31) | 52.18(19.08) |
| | After the 7th dose | 75.52(18.06) | 71.24(18.10) |
| Percent (%)[1)] of time periods in which pH≥4 during nighttime period I | Baseline | 2.98(7.75) | 3.26(8.15) |
| | After the 1st dose | 52.44(23.38) | 44.01(26.31) |
| | After the 7th dose | 71.30(26.18) | 62.30(27.01) |
| Percent (%)[2)] of time periods in which pH≥4 during nighttime period II | Baseline | 0.85(3.94) | 0.97(2.44) |
| | After the 1st dose | 77.98(23.96) | 64.69(29.16) |
| | After the 7th dose | 78.67(26.03) | 74.41(22.95) |
| Percent (%)[3)] of time periods in which pH≥4 during nighttime period III | Baseline | 4.41(10.45) | 4.59(13.04) |
| | After the 1st dose | 33.93(30.01) | 28.69(30.73) |
| | After the 7th dose | 65.77(32.40) | 52.42(36.51) |

(continued)

| Parameter | Period | Example 1 (n=41) | Comparative Example 1 (n=41) |
|---|---|---|---|
| | | Mean (SD) | Mean (SD) |
| NAB† Incidence Rate 4)t | Baseline | 41(100.00) | 41(100.00) |
| | After the 1st dose | 32(78.05) | 33(80.49) |
| | After the 7th dose | 18(43.90) | 27(65.85) |

† NAB: Nighttime acid breakthrough, defined as a state in which intragastric pH < 4 for greater than one hour during a nighttime period (22:00-06:00)

1) Nighttime period I: 22:00-06:00; 2) Nighttime period II: 21:00-01:00; 3) Nighttime period III: 01:00-05:00;

4) Proportion of patients having experienced the incidence of NAB (Nighttime Acid Breakthrough) at least one time (% of the number of patients)

After multiple doses for 7 days, the "percent of time periods in which the intragastric pH is greater than or equal to 4.0" for a 24 hour period was 75.52% in Example 1 and 71.24% in Comparative Example 1, and the corresponding parameter value for a nighttime period I (22:00-06:00) was 70.62% in Example 1 and 60.63% in Comparative Example 1. That is, compared to the tablet of Comparative Example 1, the tablet of Example 1 maintained a longer time period, in which the intragastric pH is greater than or equal to 4.0, not only for a 24 hour period but also for a nighttime period I, confirming that the tablet of Example 1 has an excellent drug efficacy maintaining effect, and this result was similar even after a single dose. Additionally, particularly during a nighttime period II (21:00-01:00) after a single dose at night, and during a nighttime period III (01:00-05:00) after multiple doses at night, intragastric pH levels were statistically significantly maintained to be greater than or equal to 4.0, which are 13.43% and 12.91% longer in Example 1 than in Comparative Example 1.

After a single dose of each of the tablets of Example 1 and Comparative Example 1, the number of subjects having NAB ("percent of the number of subjects having experienced NAB") was 32 (78.05%) in Example 1 and 33 (80.49%) in Comparative Example 1, suggesting that similar results were obtained in both cases. However, after multiple doses of each of the tablets of Example 1 and Comparative Example 1 for 7 days, the corresponding parameter value was 18 (43.90%) in Example 1 and 27 (65.85%) in Comparative Example 1, confirming that Example 1 exhibited a much lower NAB incidence rate, that is, a higher capability of maintaining nighttime drug efficacy, than Comparative Example 1.

In addition, after multiple doses for 7 days, the test drugs had a geometric mean ratio and 90% confidence interval of 1.0422 and [0.9906 - 1.0964], respectively, in the "percent of decreased integrated gastric acidity relative to the baseline over 24 hours", compared to the control drug, and were included in Log 0.80 - Log 1.25 that is the equivalence criterion defined in the statistical analysis plan.

In addition, after the first dose, the test drugs had a geometric mean ratio and 90% confidence interval of 1.0627 and [1.0198 - 1.1073], respectively, in the "percent of decreased integrated gastric acidity relative to the baseline over 24 hours", compared to the control drug, and were included in Log 0.80 - Log 1.25 that is the equivalence criterion defined in the statistical analysis protocol.

Test Example 4. In vivo Pharmacokinetic Study: PK profile (2), Part B (20 mg)

A clinical trial was carried out in the same manner as in Test Example 2, except that 20 mg of the tablet of Example 4 and Nexium ® of Comparative Example 2 were used as the investigational drugs.

A total of 48 volunteers selected as test subjects were randomized and divided into two sequence groups, and two-period crossover studies were then conducted. The investigational products corresponding to the respective sequence groups were administered to test subjects in multiple doses once a day for a total of 7 days.

All test subjects were admitted to the clinical laboratory in the afternoon two days prior to a first dose of the investigational products for each period and randomized according to the sequence groups.

[Table 11]

| Part B: 20 mg dose group (N=48) | | | |
|---|---|---|---|
| Sequence group | Number of test subjects | Period 1 | Period 2 |
| 1 | 24 | Comparative Example 2 | Example 4 |
| 2 | 24 | Example 4 | Comparative Example 2 |

There was a 7-day washout period between the first period and the second period, and investigational products of Comparative Example 2 or Example 4 were administered to all the test subjects with 150 mL water at the same time point of the morning hours. 24-hour pH monitoring and PK blood collections according to a predefined schedule were conducted in the same manner as in Test Example 2, and investigational products were administered to the test subjects at the same time points according to the groups randomized sequence groups.

After administration of the tablet of Example 4 or Comparative Example 2, blood collections planed for PK evaluation were all completed, and PK analysis was then conducted on 38 subjects with quantifiable drug concentrations.

After a single dose of each of the tablets of Example 4 and Comparative Example 2, the average concentration-time profiles of esomeprazole were evaluated for each blood collection time, and the results thereof are presented in FIG. 3. Comparative Example 2, which is for commercially available formulations, exhibited a maximum blood concentration ($C_{max}$) level at about 2 hours after drug administration, and Example 4 exhibited a maximum blood concentration ($C_{max}$) level at about 4 hours after administration. Whereas Comparative Example 2 exhibited a single peak indicating a single release profile of esomeprazole, Example 4 exhibited double peaks indicating a dual release profile of esomeprazole, and a first peak appeared about 1.75 hours after drug administration, and a second peak appeared about 4.5 hours after drug administration.

PK parameters obtained after a single dose of each of the tablets of Example 4 and Comparative Example 2 are presented in Table 12 for comparison, and geometric mean ratio and 90% confidence interval results are presented in Table 13.

[Table 12]

| Parameter | Example 4 (n=38) | | | Comparative Example 2 (n=38) | | |
|---|---|---|---|---|---|---|
| | Mean | SD | Variance coefficient (%) | Mean | SD | Variance coefficient (%) |
| $C_{max}$ (μg/L) | 499.37 | 185.00 | 37.05 | 859.48 | 320.54 | 37.30 |
| $AUC_{0-24h}$ (h × μg/L) | 1824.00 | 1117.42 | 61.26 | 1981.43 | 1317.16 | 66.48 |
| $T_{max}$ (h)[1)] | 2.25 [0.73 - 4.50] | | | 2.00 [0.98 - 4.50] | | |
| $t_{1/2}$ (h) | 1.53 0.61 40.13 | | | 1.23 | 0.64 I | 44.31 |

In the dose groups of the tablets of Example 4 and Comparative Example 2, the arithmetic mean $AUC_{0-24h}$ values were 1824.00 h×μg/L and 1981.43 h×μg/L, and the $C_{max}$ values were 499.37 μg/L and 859.48 μg/L, respectively.

In addition, compared to the Comparative Example 2-administered group, the $AUC_{0-24h}$ ($AUC_{last}$) geometric mean ratio and 90% confidence interval of the Example 4-administered group were 0.9577 and [0.08791 - 1.0433], respectively, and were included in Log 0.80 - Log 1.25, the equivalence criterion defined in the statistical analysis protocol.

The $C_{max}$ values were 237.0 μg/L and 409.2 μg/L at 0 to 3 hours and at 0 to 24 hours, respectively, after a single dose of the tablet of Example 4, while the $C_{max}$ values were 432.8 μg/L and 557.3 μg/L at 0 to 3 hours and at 0 to 24 hours, respectively, after a single dose of the tablet of Comparative Example 1.

The $AUC_{0-3h}$ and $AUC_{3-24h}$ values were 39.9 h×μg/L and 317.4 h×μg/L at 0 to 3 hours and at 3 to 24 hours, respectively, after a single dose of the tablet of Example 4. However, the $AUC_{0-3h}$ and $AUC_{3-24h}$ values were 134.8 h×μg/L and 208.1 h×μg/L at 0 to 3 hours and at 0 to 24 hours after a single dose of the tablet of Comparative Example 2, respectively.

After a single dose of the tablet of Example 4, the $AUC_{4-24h}$ value was 291.4 μg/L μg/L, while, after a single dose of the tablet of Comparative Example 2, the $AUC_{4-24h}$ value was 251.1 h×μg/L.

The $T_{max}$ (h)[1)] means the time to reach a maximum blood concentration ($C_{max}$) and is represented by a median value. The $T_{max}$ (h)[1)] value was 2.25 after a single dose of the tablet of Example 4, while the $T_{max}$ (h)[1)] value was 2.00 after a single dose of the tablet of Comparative Example 2.

Test Example 5. In vivo Pharmacodynamics Study: Therapeutic effect of NAB (2), Part B (20 mg)

A pharmacodynamics study was carried out in the same manner as in Test Example 3, except that the tablet of Example 4 or Comparative Example 2 was used as a clinical investigational drug, and pharmacodynamic (PD) analysis was conducted based on 38 subjects obtained intragastric pH data. Drug administration was started from 9 am, and intragastric pH was measured for 24 hours.

With respect to the intragastric pH, after a single dose of each of the tablets of Example 4 and Comparative Example 2 and multiple doses of 20 mg once a day for 7 days (20 mg dose of esomeprazole) "percent of time periods in which the intragastric pH is greater than or equal to 4.0" and "percent of the number of subjects having experienced NAB" are presented in Table 13.

[Table 13]

| Parameter | Period | Example 4 (n=38) | Comparative Example 2 (n=38) |
|---|---|---|---|
| | | Mean (SD) | Mean (SD) |
| Percent (%) of time periods in which pH≥4 for 24 hours | Baseline | 7.98(5.55) | 8.41(4.46) |
| | After the 1st dose | 37.94(20.05) | 32.55(20.63) |
| | After the 7th dose | 63.02(20.44) | 56.47(20.42) |
| Percent (%)[1)] of time periods in which pH≥4 during nighttime I | Baseline | 2.30(6.87) | 1.39(2.79) |
| | After the 1st dose | 27.39(28.32) | 21.31(25.91) |
| | After the 7th dose | 50.45(31.85) | 42.31(31.61) |
| Percent (%)[2)] of time periods in which pH≥4 during nighttime II | Baseline | 3.26(10.18) | 1.58(3.73) |
| | After the 1st dose | 20.17(30.79) | 12.34(25.83) |
| | After the 7th dose | 42.84(36.50) | 32.93(36.89) |
| NAB† Incidence Rate 3) | Baseline | 38(100.00) | 38(100.00) |
| | After the 1st dose | 35(92.11) | 34(89.47) |
| | After the 7th dose | 27(71.05) | 28(73.68) |

† NAB: Nighttime acid breakthrough, defined as a state in which intragastric pH < 4 for greater than one hour during a nighttime period (22:00-06:00)

1) Nighttime period I: 22:00-06:00; 2) Nighttime period II: 01:00-05:00;

3) Proportion of patients having experienced the incidence of NAB (Nighttime Acid Breakthrough) at least one time (% of the number of patients)

After multiple doses for 7 days, the "percent of time periods in which the intragastric pH is greater than or equal to 4.0" for a 24 hour period was 63.02% in Example 4 and 56.47% in Comparative Example 2, and the corresponding parameter value for a nighttime period I (22:00-06:00) was 50.45% in Example 4 and 42.31% in Comparative Example 2. That is, compared to the tablet of Comparative Example 2, the tablet of Example 4 maintained a longer time period, in which the intragastric pH is greater than or equal to 4.0, for a nighttime period I (22:00-06:00), confirming that the tablet of Example 4 has an excellent drug efficacy maintaining effect, and this result was similar even after a single dose. In addition, at nighttime II (1 a.m.-5 a.m.), Example 4 showed the effect of maintaining the intragastric pH at 4 or more for 8.83% longer after a single dose or 9.79% longer than Comparative Example 2 after multiple doses.

After a single dose, the numbers of subjects having NAB ("percent of the number of subjects having experienced NAB") was 35 (92.11%) in Example 4 and 34 (89.47%) in Comparative Example 2, which were similar in both cases, and after multiple doses for 7 days, the corresponding parameter value was 27 (71.05%) in Example 4 and 28 (73.68%) in Comparative Example 2, confirming that the capabilities of maintaining the drug efficacy during a nighttime were similarly maintained during a nighttime.

In addition, after multiple doses for 7 days, the test drugs had a geometric mean ratio and 90% confidence interval of 1.0895 and

[1.0053 - 1.1808], respectively, in "percent of decreased integrated gastric acidity relative to the baseline over 24 hours", compared to the control drug, and were included in Log 0.80 - Log 1.25 that is the equivalence criterion

defined in the statistical analysis protocol.

In addition, after the first dose, the test drugs had a geometric mean ratio and 90% confidence interval of 1.0694 and [1.0037 - 1.1394], respectively, in the "percent of decreased integrated gastric acidity relative to the baseline over 24 hours", compared to the control drug, and were included in Log 0.80 - Log 1.25 that is the equivalence criterion defined in the statistical analysis protocol.

Test Example 6. Clinical Phase III Trial: Efficacy and Safety Evaluation (40 mg)

(1) Selection of Subjects for Clinical Trials

Patients with erosive gastroesophageal reflux disease were selected as test subjects under the following selection criteria:

1) those who are 19-75 years of age at the time of consent;
2) patients who were diagnosed with erosive gastroesophageal reflux disease on screening gastroesophageal endoscopy (EGD), and classified as grade A or higher according to the LA classification system;
3) patients who experienced symptoms of heartburn or acid regurgitation within 7 days of screening; and
4) patients who voluntarily signed a written consent after being fully explained about this clinical trial.

(2) Method for Proceeding with Clinical Trials

In order to confirm non-inferiority of test drugs by comparing the efficacy after administering the test drugs to the selected test subjects with the efficacy after administering control drugs to the test subjects, clinical phase III trials were conducted in a multicenter, randomized or double blind manner.

The tablet of Example 1 (40 mg of esomeprazole) was used as a test drug, and 40 mg of Nexium® was used as a control drug. The investigational products were orally administered once a day, daily for 4+4 weeks (8 weeks in total, but administration was completed at week 4 for the subjects who had fully healed from erosion at week 4) according to the assigned test subject group.

(3) Clinical Trial Analysis Population

Among a total of 213 randomized test subjects, 211 subjects were administered investigational drugs (test drugs or control group), and by-administered group, 105 subjects administered the test drug (Example 1) and 106 subjects administered the control drug (Comparative Example 1). All of the test subject groups who were administered investigational drugs at least once after being randomized to clinical trials were used as a safety set.

Full analysis set (FAS) includes 203 subjects, except for 8 subjects who had never been subjected to primary efficacy evaluation after being randomized, and administration groups consist of 103 subjects in test drug-administered group and 100 subjects in the control drug-administered group.

Per protocol set (PPS) includes 194 subjects who completed clinical studies according to the clinical trial protocol, among FAS subjects, and by administration group, 98 subjects were in the test drug-administered group and 96 subjects were in the control drug-administered group. The test subjects with serious protocol deviations were 19 subjects, who were excluded from the PPS. Summary of clinical analysis population is presented in Table 14.

[Table 14]

| **Summary of Analysis Population** | | | |
|---|---|---|---|
| Number of population (N) (%) | Test drug-administered group (N=107) | Control drug-administered group (N=106) | Total (N=213) |
| Total enrollment | 107 (100.00%) | 106 (100.00%) | 213 (100.00%) |
| Safety set (SS) | 105 (98.13%) | 106 (100.00%) | 211 (99.06%) |
| Full analysis set (FAS) | 103 (96.26%) | 100 (94.34%) | 203 (95.31%) |
| Per protocol set (PPS) | 98 (91.59%) | 96 (90.57%) | 194 (91.08%) |

(4) Efficacy and Safety Evaluation Method

For primary efficacy evaluation, a per protocol set (PPS) was used as a main analysis set, and a full analysis set (FAS) was used as a subsidiary analysis set. Secondary efficacy evaluation was performed on both analysis sets.

The complete healing rate of erosions was defined as the proportion of subjects who were determined, among all test subjects, to be "Not Present" according to the Los Angeles (LA) classification of erosions, as examined by gastroesophageal endoscopy, after administration of investigational products.

1) Primary Efficacy Evaluation Method

At week 8, primary efficacy was evaluated on the basis of the complete healing rate of erosions, as examined by gastroesophageal endoscopy. However, when erosions were completely healed after 4 weeks of treatment and completed normally on gastroesophageal endoscopy, the subjects were included in completely healed subjects. In order to test the non-inferiority of the test drug-administered group compared to the control drug-administered group, a 95% confidence interval was calculated by the normal approximation method. However, when the number of cells with an expected frequency of less than 5 exceeds 20% of total cells, a 95% confidence interval was calculated by an exact method. When the lower limit of the 95% two-sided confidence interval for the difference between two groups (test group - control group) in the complete healing rate of erosions was greater than the non-inferiority margin, that is, - 10%, the test group was determined to be non-inferior to the control group.

2) Secondary Efficacy Evaluation Method

At week 4, the complete healing rate of erosions, as examined by gastroesophageal endoscopy, was presented, and a 95% confidence interval was calculated using the normal approximation method. However, when the number of cells with an expected frequency of less than 5 exceeds 20% of total cells, a 95% confidence interval was calculated by an exact method.

Main items of secondary efficacy evaluation are as follows:

① complete healing rate of erosions on gastroesophageal endoscopy at week 4;
② complete resolution rate of each symptom in GERD at week 4 and week 8 (Here, the complete resolution rate of each symptom is defined as a case corresponding to 'Score 0' in the classification of severity for 7 days. In addition, the time point of week 8 is evaluated based on subjects who have undergone clinical trials up to 8 weeks because erosion has not been completely healed at week 4.);
③ proportion of heartburn-free days, acid regurgitation-free days for one day after 1, 2, 4, or 8 week treatment;
④ proportion of heartburn-free nights, acid regurgitation-free nights during a nighttime after 1, 2, 4, or 8 week treatment;
⑤ time to sustained resolution of heartburn, acid regurgitation for one day; and
⑥ time to sustained resolution of nocturnal heartburn, nocturnal acid regurgitation during a nighttime.

3) Items of safety evaluation are as follows:

① adverse reactions; and
② physical examination, vital signs, clinical laboratory tests (hematology test, blood chemistry test, urinalysis), or electrocardiogram.

(5) efficacy and safety evaluation results

1) Primary Efficacy Evaluation Results

For primary efficacy evaluation, PPS was used as a main analysis set, and FAS was used as a subsidiary analysis set, and the number (N) of completely healed subjects, the complete healing rate (%) and 95% confidential interval were presented. At week 8 after administration of investigational products, the complete healing rates, as examined by gastroesophageal endoscopy, are indicated in Tables 15 and 16.

[Table 15]

| Complete Healing Rate in PPS at week 8 | | |
|---|---|---|
| Healing Rate | Test drug (N=98) | Control drug (N=96) |
| N (%) | 96(97.96%) | 93(96.88%) |
| Difference | 1.08 | - |
| 95% CI for Normal approximation | (-4.46,7.21) | - |
| p-value | <0.0001 | - |

[Table 16]

| Complete Healing Rate in FAS at week 8 | | |
|---|---|---|
| Healing Rate | Test drug (N=103) | Control drug (N=100) |
| N (%) | 101(98.06%) | 95(95.00%) |
| Difference | 3.06 | - |
| 95% CI for Normal approximation | (-2.54, 9.72) | - |
| p-value | <0.0001 | - |

As indicated in Table 15, as primary evaluation parameter, on the basis of PPS, the complete healing rate (% (N)) of erosions at week 8, as examined by gastroesophageal endoscopy, was 97.96% (96/98) in the test drug group-administered group and 96.88% (93/96) in the control drug-administered group, suggesting that the complete healing rate was 1.08% higher in the test drug-administered group, and the 95% two-sided confidence interval of the difference between the two groups was [-4.46, 7.21].

In addition, as indicated in Table 16, on the basis of FAS, the complete healing rates (% (N)) of erosions at week 8, as examined by gastroesophageal endoscopy, was 98.06% (101/103) in the test drug-administered group and 95.00% (95/100) in the control drug-administered group, suggesting that the complete healing rate was 3.06% higher in the test drug-administered group, and the 95% two-sided confidence interval of the difference between the two groups was [-2.54, 9.72].

Overall, since the lower limits of the 95% two-sided confidence interval of the difference between the two groups in PPS and FAS were -4.46% and -2.54%, respectively, which were larger than the non-inferiority margin, that is, -10%, in both analysis sets, it was proved that the test drug-administered group was non-inferior to the control drug-administered group with respect to the complete healing rate at week 8 after administration.

2) Secondary Efficacy Evaluation Results

At week 4, on the basis of PPS, the complete healing rate (% (N)) of erosions, as examined by gastroesophageal endoscopy, was 91.84% (90/98) in the test drug-administered group and 91.67% (88/96) in the control drug-administered group, suggesting that the complete healing rate was 0.17% higher in the test drug-administered group, and the 95% two-sided confidence interval of the difference between the two groups was [-7.57, 7.91].

On the basis of FAS, the complete healing rates (% (N)) of erosions at week 4, as examined by gastroesophageal endoscopy, was 92.23% (95/103) in the test drug-administered group and 89.00% (89/100) in the control drug-administered group, suggesting that the complete healing rate was 3.23% higher in the test drug-administered group, and the 95% two-sided confidence interval of the difference between the two groups was [-4.79, 11.25].

The lower limits of the 95% two-sided confidence interval for PPS and FAS were -7.57% and -4.79%, respectively, which were larger than the non-inferiority margin, that is, -10% for in both analysis sets. In conclusion, this clinical trial demonstrated that the complete healing rate satisfies the non-inferiority of the test drug-administered group at week 4 as well as at week 8, after administration, compared to the control drug-administered group.

[Table 17]

| Complete Healing Rate in PPS at week 4 | | |
|---|---|---|
| Healing Rate | Test drug (N=98) | Control drug (N=96) |
| N (%) | 90(91.84%) | 88(91.67%) |

(continued)

| Complete Healing Rate in PPS at week 4 | | |
|---|---|---|
| Difference | 0.17 | - |
| 95% CI for Normal approximation | (-7.57, 7.91) | - |
| p-value | 0.0050 | - |

[Table 18]

| Complete Healing Rate in FAS at week 4 | | |
|---|---|---|
| Healing Rate | Test drug (N=103) | Control drug (N=100) |
| N (%) | 95(92.23%) | 89(89.00%) |
| Difference | 3.23 | - |
| 95% CI for Normal approximation | (-4.79, 11.25) | - |
| p-value | 0.0006 | - |

In PPS, the complete resolution rates (%) of all of four gastroesophageal reflux disease symptoms was 51.02% (N, 50/98) in the test drug-administered group and 51.04% (N, 49/96) in the control drug-administered group at week 4, and was 37.50% (N, 3/8) in the test drug-administered group and 25.00% (N, 2/8) in the control drug-administered group at week 8. Here, the four gastroesophageal reflux disease symptoms mean heartburn, acid regurgitation, dysphagia, and epigastric pain.

In PPS, the proportions (%, Mean (SD)) of heartburn-free days for one day were: after 1-week treatment, 57.14% (39.24%) in the test drug group and 50.74% (37.36%) in the control drug-administered group; after 2-week treatment, 62.35% (35.80%) in the test drug group and 61.01% (34.04%) in the control drug-administered group; and after 4-week treatment, 70.28% (31.76%) in the test drug group and 72.34% (28.19%) in the control drug-administered group. Overall, at week 1, 2 after initial administration, the proportions of heartburn-free days for one day tended to be higher in the test drug-administered group than in the control drug-administered group, showing the largest difference (6.40%) at week 1. Additionally, at week 1, the proportion of heartburn-free nights during a nighttime was 75.80% (34.29%) in the test drug-administered group and 74.26% (34.97%) in the HGP1705-administered group.

In PPS, the proportions (%, Mean (SD)) of acid regurgitation-free days for one day were: after 1-week treatment, 62.24% (40.88%) in the test drug group and 56.10% (42.09%) in the control drug-administered group; after 2-week treatment, 67.09% (36.68%) in the test drug group and 65.77% (36.77%) in the control drug-administered group; and after 4-week treatment, 65.77% (36.77%) in the test drug-administered group and 73.71% (30.49%) in the control drug-administered group. Overall, at week 1, 2 after initial administration, the proportions of acid regurgitation-free days for one day tended to be higher in the test drug-administered group than in the control drug-administered group, showing the largest difference (6.14%) at week 1. Additionally, at week 1, the proportion of acid regurgitation-free nights during a nighttime was 78.43% (33.72%) in the HGP1705-administered group and 76.64% (33.88%) in the HGP1705-administered group. In PPS, the time (Median, [95% CI]) to sustained resolution of heartburn symptoms for one day was 7.00 days [3.00, 15.00] in the test drug-administered group and 8.00 days [6.00, 11.00] in the control drug-administered group. In the case of acid regurgitation symptoms, the time to sustained resolution thereof was 5.00 days [2.00, 9.00] in the test drug-administered group and 6.00 days [3.00, 9.00] in the control drug-administered group, showing that the time to reach symptom resolution was one day faster in the test drug-administered group.

Additionally, the time (Median, [95% CI]) to sustained resolution of heartburn symptoms during a nighttime was 1.00 day [1.00, 3.00] in the test drug-administered group and 1.00 day [1.00, 3.00] in the control drug-administered group. In the case of acid regurgitation symptoms, the time to sustained resolution thereof was 1.00 day [1.00, 2.00] in the test drug-administered group and 1.00 day [1.00, 3.00] in the control drug-administered group.

3) Safety Evaluation Results

As a result of safety evaluation, no clinically significant adverse reactions occurred in both of the test drug-administered group and the control drug-administered group.

As a result of the above efficacy and safety evaluation, it was proved that when 40 mg of the test drug was administered, the therapeutic effect of erosive gastroesophageal reflux disease, as confirmed by gastroesophageal endoscopy, was non-inferior to a case when the control drug was administered, at both of week 4 and week 8, and it was also confirmed that the safety level was good in the test drug-administered group. Therefore, it is considered that

the administration of the test drug is effective and safe in erosive gastroesophageal reflux diseases.

Test Example 7. Dietary Effect Evaluation - Pharmacokinetic/Pharnacodynamics Study: PK/PD Profile (40 mg)

To investigate the effects of food on pharmacokinetic (PK) and pharmacodynamic (PD) properties in healthy adult male volunteer subjects after oral administration of the tablet of Example 1, randomized, open-label, single dose and crossover studies were conducted.

A total of 24 volunteers selected as test subjects were randomized and divided into two sequence groups, and two-period crossover studies were then conducted. The test subjects were administered in a single dose of the investigational drug once a day under the dietary conditions (fasted or high-fat diet) corresponding to each sequence group.

All test subjects were admitted to the clinical laboratory in the afternoon two days prior to a first dose of the investigational products for each period and randomized according to the sequence groups.

[Table 19]

| Sequence group | Number of test subjects | Period 1 | Period 2 |
|---|---|---|---|
| 1 | 12 | Fasted | Fed (high-fat diet) |
| 2 | 12 | Fed (high-fat diet) | Fasted |

A 7-day washout period was given between the first period and the second period, and investigational products of Example 1 were administered to all of the test subjects with 150 mL water at the same time point of the morning hours after food intake.

One day prior to a first dose, the test subjects were subjected to 24-hour pH monitoring in the fasted condition or after intake of a high-fat diet for baseline evaluation at given time points. According to the group assigned to the first administration day of the investigational products, the test subjects were administered the test drugs of Example 1 in a fasted condition or after intake of a high-fat diet (900 kcal or more, containing 35% or more fat) within 20 minutes, followed by conducting 24-hour pH monitoring and pharmacokinetic blood collections according to the predefined schedule. The next day, the test subjects were subjected to safety, PK and PD evaluation tests (including pH monitoring) according to the predefined schedules blood collection prior to administration, and then discharged after completing all hospitalization study schedules.

After administration of the tablet of Example 1 according to the dietary conditions, all scheduled blood collection for pharmacokinetic evaluation was completed, and pharmacokinetic analysis was performed on 23 subjects with quantifiable drug concentrations.

The average concentration-time pattern of esomeprazole at each blood sampling time point after a single dose of a formulation of Example 1 according to dietary conditions is presented in FIG. 5. FIG. 5 shows the average concentration-time pattern of esomeprazole at each blood sampling time point after a single dose of a formulation of Example 1 according to dietary conditions (high-fat diet or fasted). When the drug was administered in the fasted condition, the maximum blood concentration appeared at about 4-hour time point, and when the drug was administered after intake of a high-fat diet, the maximum blood concentration appeared at about 5.5-hour time point. In addition, when administered the formulation of Example 1 after intake of a high-fat diet or in a fasted condition, the arithmetic mean of $AUC_{0-24h}$ was 1861.81 and 2700.77 h×μg/L, respectively, and $C_{max}$ was 460.30 and 768.43 μg/L, respectively.

[Table 20]

| Parameter | High-fat diet ( n=23) | | | Fasted (n=23) | | |
|---|---|---|---|---|---|---|
| | Mean | SD | Variance coefficient (%) | Mean | SD | Variance coefficient (%) |
| $C_{max}$ (μg/L) | 460.30 | 329.19 | 71.51 | 768.43 | 283.37 | 36.88 |
| $AUC_{0-24h}$ (h × μg/L) | 1881.81 | 1584.36 | 85.10 | 2700.77 | 1720.50 | 63.70 |
| $T_{max}$ (h)[1)] | 5.50 [2.00 - 10.00] | | | 4.00 [1.00 - 5.00] | | |
| $t_{1/2}$ (h) | 1.55 | 0.73 | 47.17 | 1.35 | 0.49 | 36.21 |

After administration of the tablet of Example 1 according to the dietary conditions, 24-hour pH monitoring was all completed, and pharmacodynamic (PD) analysis was performed on 21 subjects who offered evaluable intragastric pH data.

After administration of the investigational product, the geometric mean of the percent (%) of decreased integrated

gastric acidity relative to the baseline over 24 hours was 67.82% after intake of a high-fat diet and 71.95% in a fasted condition. The geometric mean ratio and 90% confidence interval were 0.9474 and [0.8750-1.0258], which were included in Log 0.80 - Log 1.25, the equivalence criterion, and thus it was confirmed that the 24 hour-pharmacodynamic effects were equivalent in a single dose of the tablet of Example 1 in a fasted condition and after intake of a high-fat diet. In addition, the time (%) in which the intragastric pH is maintained to be 4.0 or higher was 35.00% after intake of a high-fat diet and 35.99% in a fasted condition, and a difference between two groups was not statistically significant (p=0.7868). FIG. 6 shows graphs of integrated gastric acidity according to dietary conditions (high-fat diet or fasted) and time. FIG. 7 shows a graph of mean intragastric pH according to dietary conditions (high-fat diet or fasted) and time after a single dose of formulation of Example 1.

In the present specification, "integrated gastric acidity" may be calculated from intragastric pH measurements, and may be used to quantify gastric acidity over time. The integrated gastric acidity was calculated by using the following formulas:

- Acid concentration (mmol/L) = $1000 \times 10^{-pH}$
- Integrated acidity $(\text{mmol x h/L}) = \sum \frac{\text{acid in mmol/L at time "t"} + \text{acid in mmol/L at time "}t_{-1}\text{"}}{2}$ $\times$ time in second
- Percent decrease from baseline in integrated gastric acidity for time interval after 1st dose (%) = $[\frac{\text{(Baseline - or } 1^{st} \text{ dose}}{\text{Baseline}}] \times 100$

[Table 21]

| | N | High-fat diet (Fed) | Fasted | Geometric Mean Ratio [90% CI] |
|---|---|---|---|---|
| Percent (%) of decreased integrated gastric acidity | 21 | 67.82 | 71.95 | 0.9474 [0.8750-1.0258] |

[Table 22]

| Parameter | Period | High-fat diet (n=21) | Fasted (n=21) |
|---|---|---|---|
| | | Mean (SD) | Mean (SD) |
| Percent (%) of time periods in which pH≥4 for 24 hours | Baseline<br>After the 1st dose | 14.57(11.40)<br>35.00(16.84) | 7.88(3.77)<br>35.99(10.60) |

For reference, in the previously reported dietary impact evaluation study results, it is known that a single dose of the esomeprazole formulation (Comparative Example 1), developed as a single-release formulation, reduces AUC and $C_{max}$ by about 44% and about 66% in the high-fat diet group, compared to the fasted group, and delays $T_{max}$ by about 2 hours (Mark B. Sostek et al, Effect of timing of dosing in relation to food intake on the pharmacokinetics of esomeprazole. Br J Clin Pharmacol 2007; 64:3 386-390). Compared with these results, the pharmacokinetic results of Example 1 having the characteristics of the dual-release formulation confirmed that the degrees in which AUC and $C_{max}$ were reduced due to a high-fat diet were about 31% and 40%, confirming that the decreases were less than those of Comparative Example 1, and the delay time for drug absorption was also reduced to 1.5 hours. In addition, in the pharmacodynamic effect evaluation results of Example 1 according to the dietary conditions, the percent (%) of decreased integrated gastric acidity were biologically equivalent, and there was also no statistically significant difference in the time (%) in which the intragastric pH is maintained to be 4.0 or higher. Therefore, when such pharmacokinetic/pharmacodynamic evaluation results are combined, it is determined that the dietary effect on the gastric acid secretion inhibitory ability of Example 1 is not significant, and the overall effect on the dietary effect is less than that of Comparative Example 1.

Although specific parts of the present disclosure have been described above in detail, it will be apparent to those of ordinary skill in the art that these specific descriptions are only preferred embodiments, and the scope of the present invention is not limited thereby. Accordingly, the substantial scope of the present disclosure will be defined by the appended claims and their equivalents.

## Claims

1. A pharmaceutical composition comprising:
an immediate-release enteric-coated tablet comprising esomeprazole or a pharmaceutically acceptable salt thereof

as an active ingredient and a sustained-release enteric-coated tablet comprising esomeprazole or a pharmaceutically acceptable salt thereof as an active ingredient, the pharmaceutical composition having the following release characteristics:

(i) the maximum blood concentration ($C_{max\ 0\text{-}3h}$) of the active ingredient in blood within 0 to 3 hours after administration is 40% to 80% of the maximum concentration ($C_{max\ 0\text{-}24h}$) of the active ingredient within 0 to 24 hours after administration,
(ii) a time ($T_{max}$) to reach the maximum blood concentration within 0 to 24 hours is 2 hours after administration; and
(iii) $AUC_{3\text{-}24h}$ is at least 5 times $AUC_{0\text{-}3h}$.

2. The pharmaceutical composition of claim 1, wherein the $AUC_{0\text{-}24h}$ of the composition is 70 to 130% compared to $AUC_{0\text{-}24h}$ of the esomeprazole single-release formulation of the same dose.

3. The pharmaceutical composition of claim 1, wherein the $AUC_{4\text{-}24h}$ of the composition is 120 to 170% compared to $AUC_{4\text{-}24h}$ of the esomeprazole single-release formulation of the same dose.

4. The pharmaceutical composition of claim 1, wherein the time taken for the composition to reach the maximum blood concentration ($C_{max\ 0\text{-}24h}$) within 0 to 24 hours is 3 to 7 hours after administration.

5. The pharmaceutical composition of claim 1, wherein the time ($T_{max}$) taken for the composition to reach the maximum blood concentration within 0 to 24 hours is 3 hours after administration.

6. The pharmaceutical composition of claim 1, wherein the composition is a capsule filled with a mixture of the immediate-release enteric-coated tablet and the sustained-release enteric-coated tablet.

7. The pharmaceutical composition of claim 6, wherein the immediate-release enteric-coated tablet and the sustained-release enteric-coated tablet are multi-unit spheroidal tablets (MUSTs).

8. The pharmaceutical composition of claim 1, wherein the composition includes the immediate-release enteric-coated tablet and the sustained-release enteric-coated tablet in a ratio of 1:1.

9. The pharmaceutical composition of claim 1, wherein each of the immediate-release enteric-coated tablet and the sustained-release enteric-coated tablet comprises a core comprising an active ingredient, and the core further comprises one or more excipients selected from a diluent, a binder, a disintegrant, a lubricant, a surfactant, an antioxidant, a preservative, and a stabilizer.

10. The pharmaceutical composition of claim 8, wherein the immediate-release enteric-coated tablet and sustained-release enteric-coated tablet include an inner coating layer formed on each core, and the inner coating layer includes one or more coating bases selected from the group consisting of hydroxypropyl methylcellulose (HPMC), polyvinylpyrrolidone (PVP), low-substituted hydroxypropyl cellulose (HPC-L), starch, gelatin, ethyl cellulose, and any combination thereof.

11. The pharmaceutical composition of claim 9, wherein the immediate-release enteric-coated tablet comprises an immediate-release enteric coating layer formed on the inner coating layer, and the sustained-release enteric-coated tablet comprises a sustained-release enteric coating layer formed on the inner coating layer.

12. The pharmaceutical composition of claim 1, wherein the composition comprises esomeprazole or a pharmaceutically acceptable salt thereof as an active ingredient in an amount of 10 to 50 mg per unit dosage form.

13. The pharmaceutical composition of claim 1, wherein the composition is administered once a day.

14. The pharmaceutical composition of claim 1, wherein the composition may be for use in preventing or treating nocturnal acid breakthrough.

15. The pharmaceutical composition of claim 1, wherein the release characteristics of the composition appears in a fasted condition or before having a meal when the composition is administered.

# FIG. 1

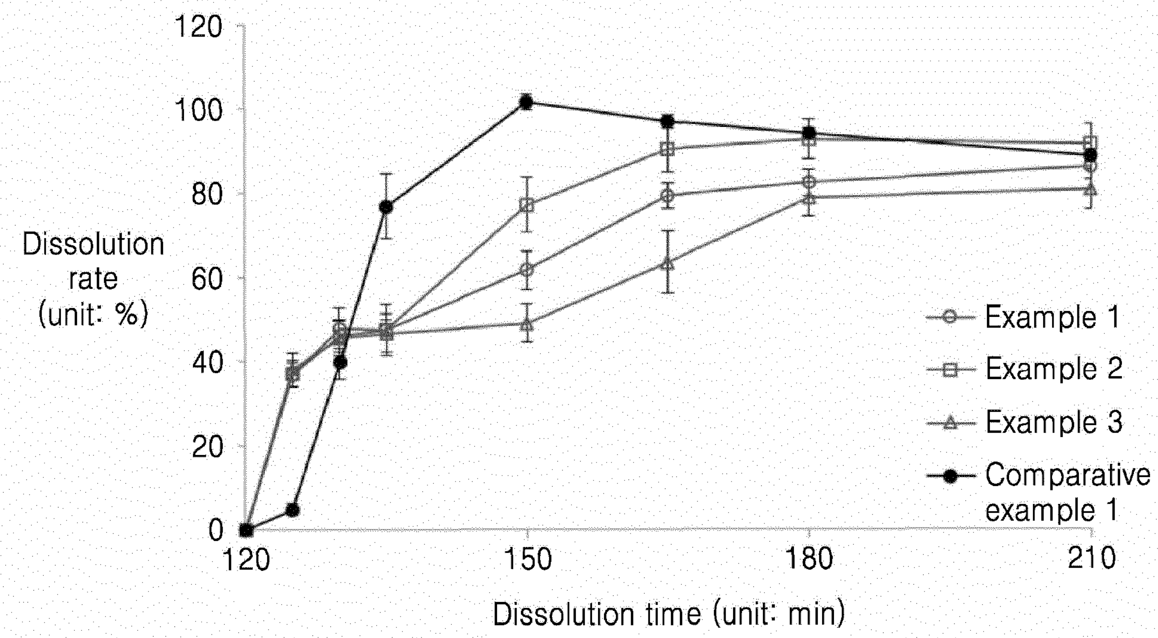
120
100
80
60
40
20
0
Dissolution rate (unit: %)
120
150
180
210
Dissolution time (unit: min)
Example 1
Example 2
Example 3
Comparative example 1

# FIG. 2

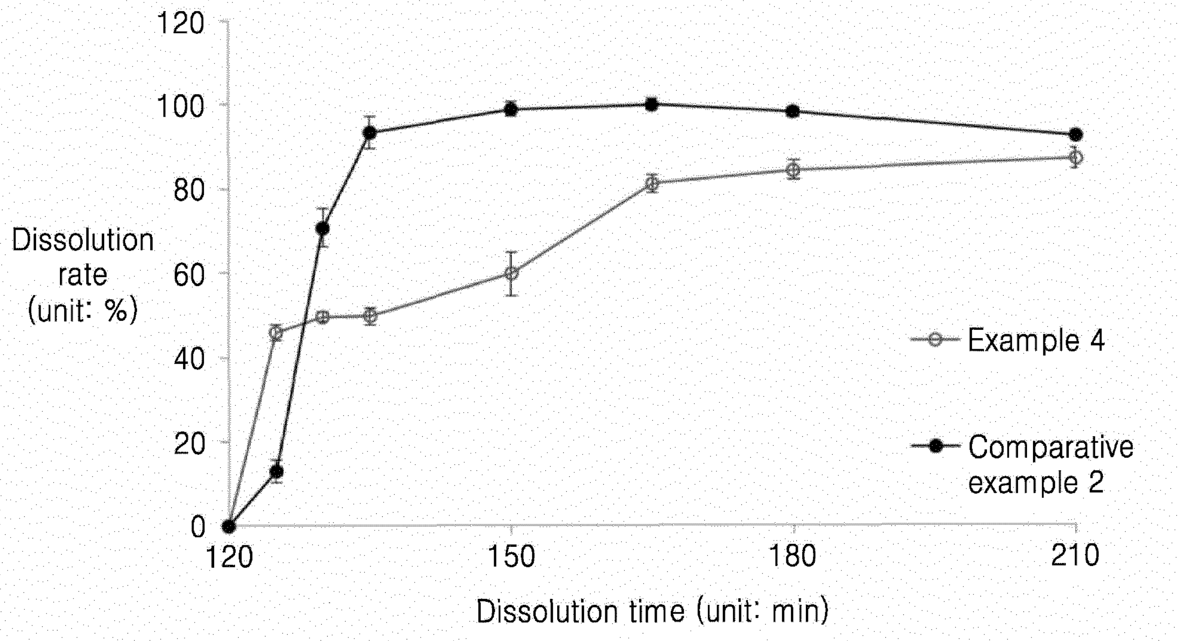
120
100
80
60
40
20
0
Dissolution rate (unit: %)
120
150
180
210
Dissolution time (unit: min)
Example 4
Comparative example 2

FIG. 3

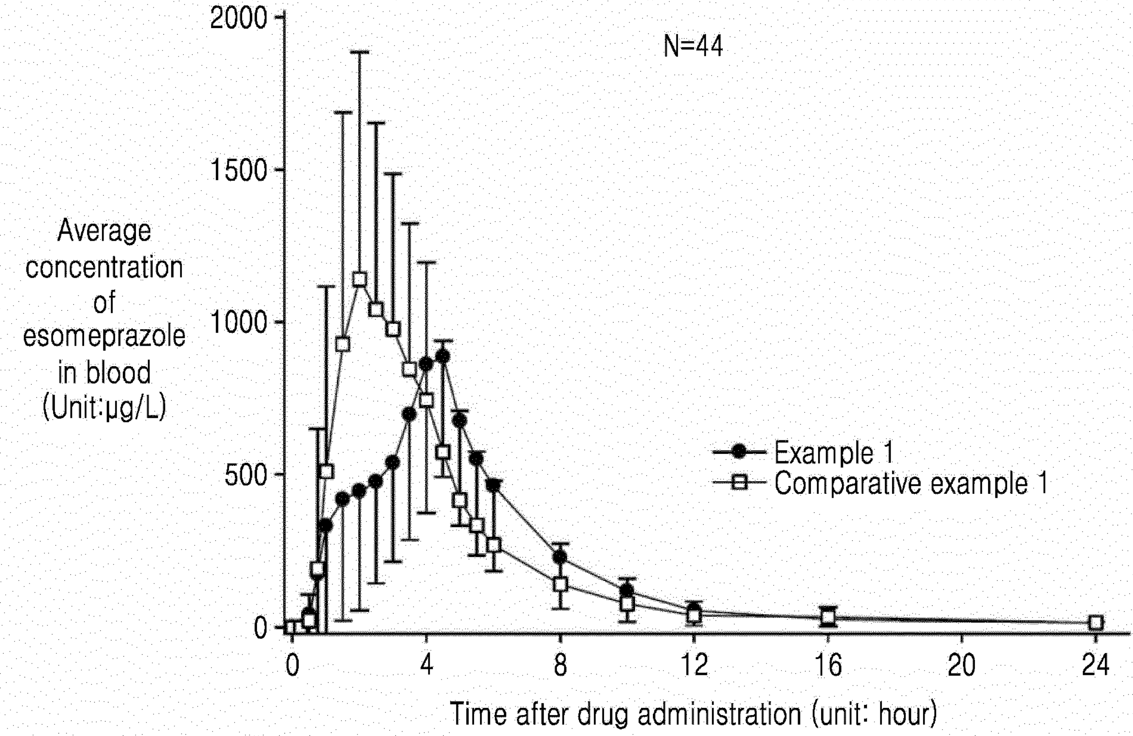
N=44
Average concentration of esomeprazole in blood (Unit:μg/L)
2000
1500
1000
500
0
0
4
8
12
16
20
24
Time after drug administration (unit: hour)
Example 1
Comparative example 1

FIG. 4

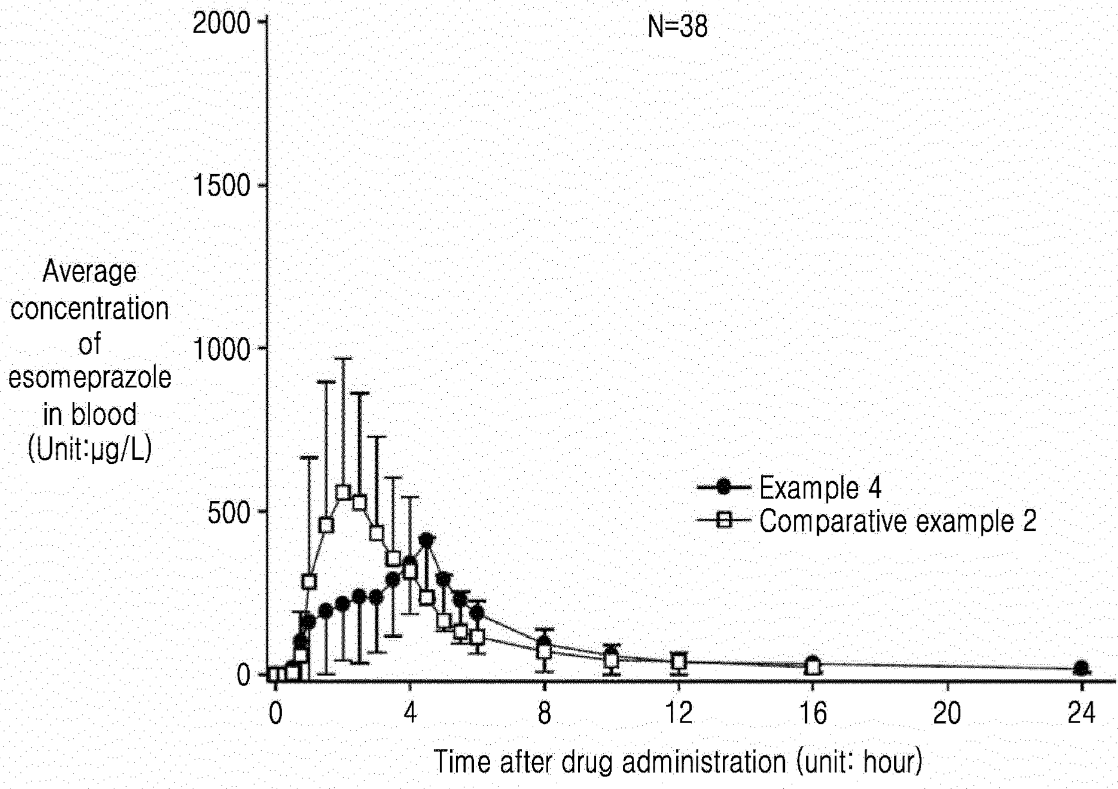

N=38
Average concentration of esomeprazole in blood (Unit:μg/L)
2000
1500
1000
500
0
Example 4
Comparative example 2
0
4
8
12
16
20
24
Time after drug administration (unit: hour)

FIG. 5

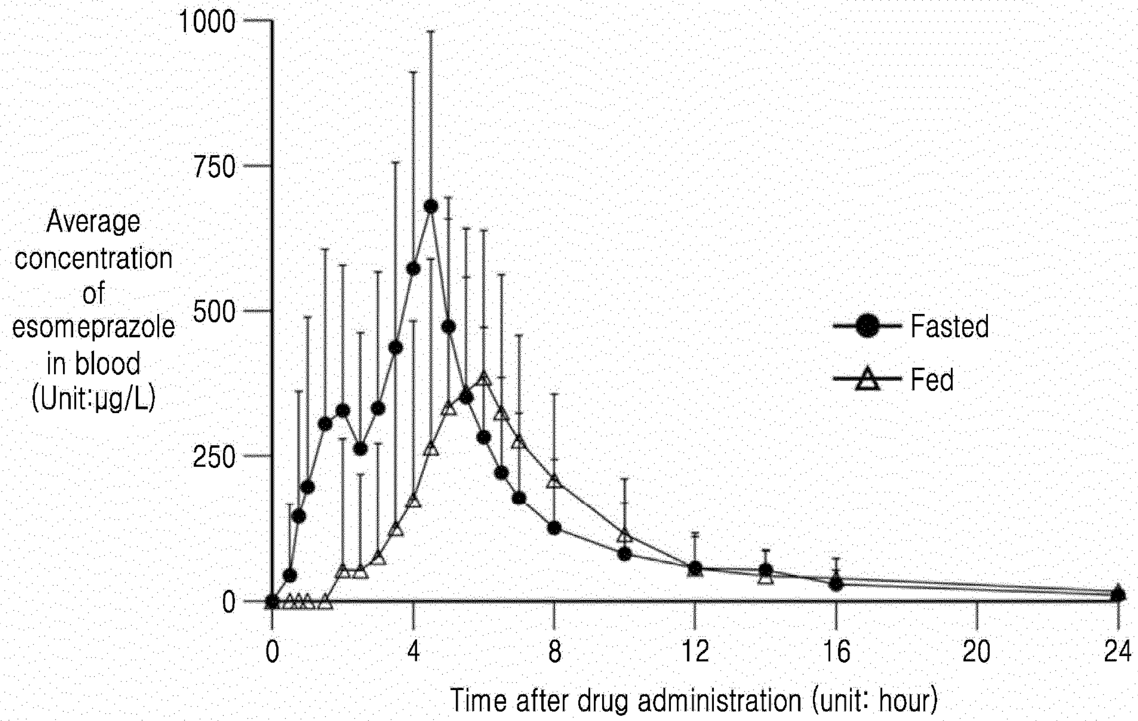
1000
750
500
250
0
Average concentration of esomeprazole in blood (Unit:μg/L)
Fasted
Fed
0
4
8
12
16
20
24
Time after drug administration (unit: hour)

FIG. 6

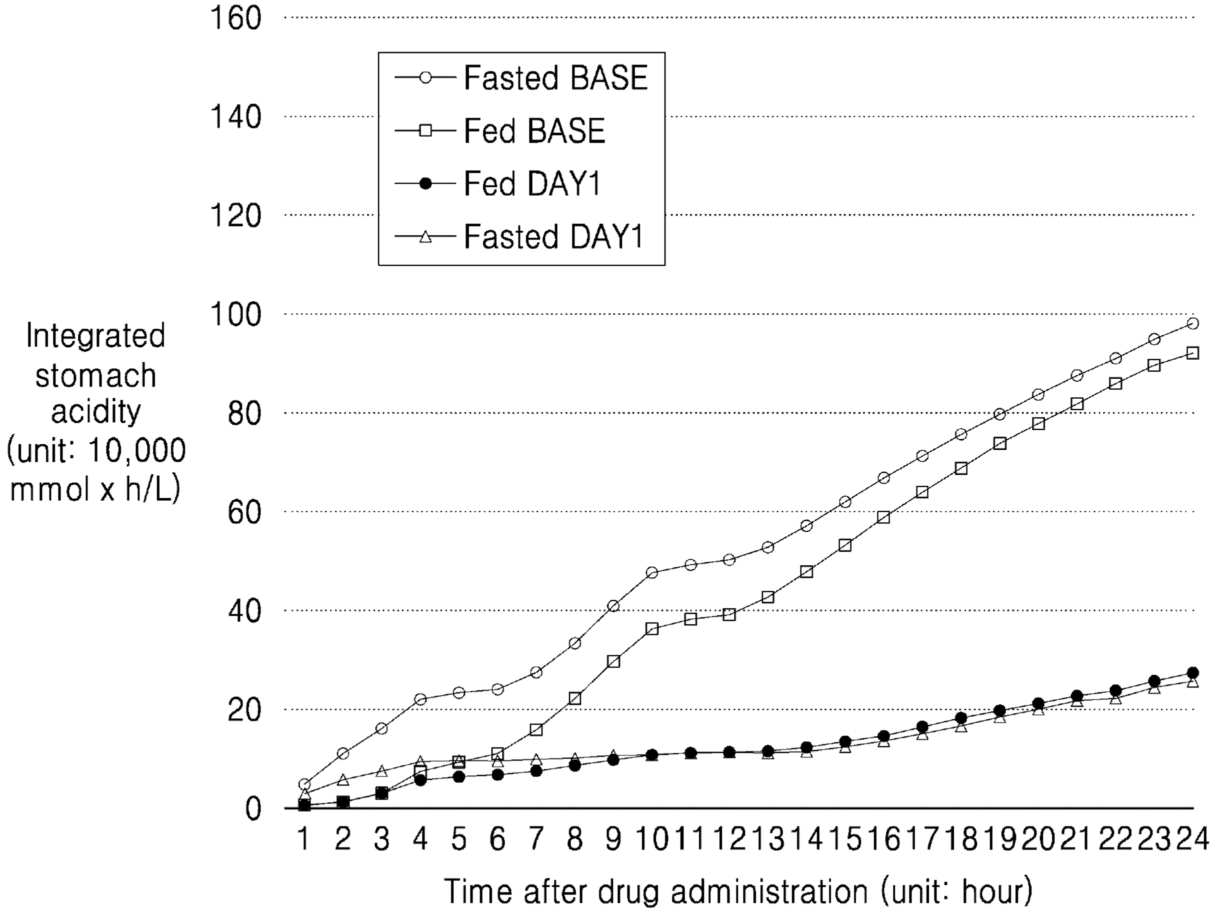
160
140
120
100
80
60
40
20
0
Fasted BASE
Fed BASE
Fed DAY1
Fasted DAY1
Integrated stomach acidity (unit: 10,000 mmol x h/L)
1 2 3 4 5 6 7 8 9 10 11 12 13 14 15 16 17 18 19 20 21 22 23 24
Time after drug administration (unit: hour)

# FIG. 7

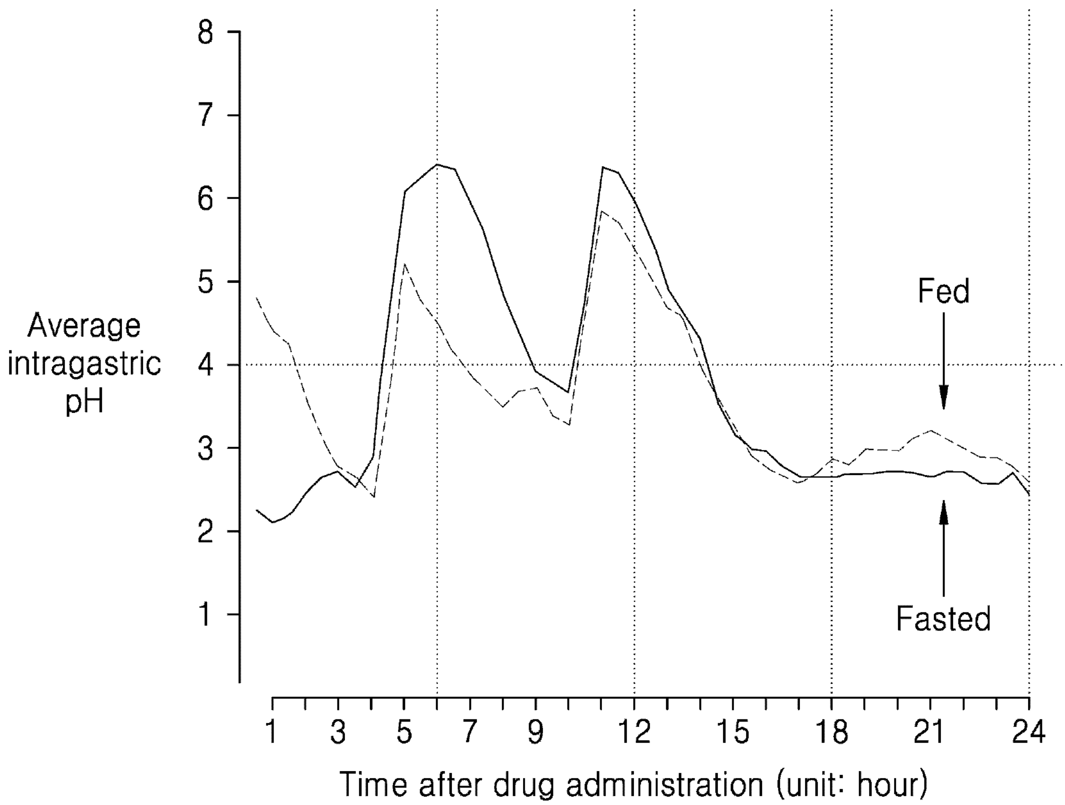
8
7
6
5
4
3
2
1
Average intragastric pH
Fed
Fasted
1
3
5
7
9
12
15
18
21
24
Time after drug administration (unit: hour)

## INTERNATIONAL SEARCH REPORT

International application No.
PCT/KR2020/004474

**A. CLASSIFICATION OF SUBJECT MATTER**

*A61K 9/48(2006.01)i, A61K 9/28(2006.01)i, A61K 31/4439(2006.01)i, A61P 1/04(2006.01)i*

According to International Patent Classification (IPC) or to both national classification and IPC

**B. FIELDS SEARCHED**

Minimum documentation searched (classification system followed by classification symbols)
A61K 9/48; A61K 31/415; A61K 31/4178; A61K 31/4439; A61K 9/16; A61K 9/28; A61K 9/52; A61P 1/04

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: esomeprazole, immediate release, extended release, release control, maximum plasma concentration (Cmax)

**C. DOCUMENTS CONSIDERED TO BE RELEVANT**

| Category* | Citation of document, with indication, where appropriate, of the relevant passages | Relevant to claim No. |
|---|---|---|
| X | KR 10-2018-0046682 A (HANMI PHARM. CO., LTD.) 09 May 2018<br>See abstract, claims 1, 3, 8, 9, paragraphs [0022]-[0023], [0027]-[0028], [0033], [0054]-[0061], [0104], [0195], figure 1. | 1-15 |
| A | KR 10-2017-0061219 A (KOREA UNITED PHARM. INC.) 05 June 2017<br>See the entire document. | 1-15 |
| A | KR 10-2010-0078462 A (HANMI PHARM. CO., LTD.) 08 July 2010<br>See the entire document. | 1-15 |
| A | KR 10-2018-0011624 A (HANMI PHARM. CO., LTD.) 02 February 2018<br>See the entire document. | 1-15 |
| A | KR 10-2013-0115864 A (HANMI PHARM. CO., LTD.) 22 October 2013<br>See the entire document. | 1-15 |

☐ Further documents are listed in the continuation of Box C.

☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 JUNE 2020 (30.06.2020) | **30 JUNE 2020 (30.06.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2020/004474**

| Patent document cited in search report | Publication date | Patent family member | Publication date |
|---|---|---|---|
| KR 10-2018-0046682 A | 09/05/2018 | CN 109890372 A | 14/06/2019 |
| | | EP 3513784 A1 | 24/07/2019 |
| | | JP 2019-532960 A | 14/11/2019 |
| | | US 2019-0247316 A1 | 15/08/2019 |
| | | WO 2018-080104 A1 | 03/05/2018 |
| KR 10-2017-0061219 A | 05/06/2017 | KR 10-1869406 B1 | 24/07/2018 |
| KR 10-2010-0078462 A | 08/07/2010 | KR 10-1084659 B1 | 22/11/2011 |
| KR 10-2018-0011624 A | 02/02/2018 | CN 109789102 A | 21/05/2019 |
| | | EP 3488845 A1 | 29/05/2019 |
| | | JP 2019-523258 A | 22/08/2019 |
| | | KR 10-1907116 B1 | 11/10/2018 |
| | | US 2019-0240209 A1 | 08/08/2019 |
| | | WO 2018-021772 A1 | 01/02/2018 |
| KR 10-2013-0115864 A | 22/10/2013 | CN 104220050 A | 17/12/2014 |
| | | CN 110051642 A | 26/07/2019 |
| | | EP 2836207 A1 | 18/02/2015 |
| | | EP 2836207 B1 | 19/02/2020 |
| | | JP 2015-517998 A | 25/06/2015 |
| | | JP 6129295 B2 | 17/05/2017 |
| | | KR 10-1378973 B1 | 28/03/2014 |
| | | US 2015-0098992 A1 | 09/04/2015 |
| | | WO 2013-154390 A1 | 17/10/2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20130115864


### Non-patent literature cited in the description


- **REZA SHAKER et al.** *THE AMERICAN JOURNAL OF GASTROENTEROLOGY,* vol. 98 (7), 1487-1494
- Mechanism of action and usage of Proton Pump Inhibitors. *Journal of the Korean Society of Gastroenterology,* 2006, vol. 48, 4-8
- **MARK B. SOSTEK et al.** Effect of timing of dosing in relation to food intake on the pharmacokinetics of esomeprazole. *Br J Clin Pharmacol,* 2007, vol. 64 (3), 386-390